# EUROPÄISCHE PATENTSCHRIFT

(11) **EP 2 923 234 B1**
(45) Veröffentlichungstag und Bekanntmachung des Hinweises auf die Patenterteilung: **28.12.2016**
(21) Anmeldenummer: 13799207.9
(22) Anmeldetag: 21.11.2013
(51) Int. Cl.: G02C 7/10, A61F 9/02, A61F 9/06

(54) **VERFAHREN ZUM REDUZIEREN DER VON EINEM TRÄGER EINER BRILLE WAHRNEHMBAREN HELLIGKEIT MINDESTENS EINES OBJEKTS SOWIE BLENDSCHUTZBRILLE**
METHOD FOR REDUCING THE LIGHT INTENSITY OF AT LEAST ONE OBJECT PERCEIVABLE BY A SPECTACLE WEARER, AND ANTI-GLARE SPECTACLES
PROCÉDÉ DE RÉDUCTION DE LA LUMINOSITÉ D'AU MOINS UN OBJET PERCEPTIBLE PAR UN PORTEUR DE LUNETTES, AINSI QUE LUNETTES ANTI-ÉBLOUISSEMENT

(30) Priorität: 22.11.2012 US 201261729396 P; 23.11.2012 DE 102012022914
(43) Veröffentlichungstag der Anmeldung: 30.09.2015
(73) Patentinhaber: Röder, Jürgen, 78467 Konstanz (DE)
(72) Erfinder: Röder, Jürgen, 78467 Konstanz (DE)
(74) Vertreter: Fugmann, Winfried
(86) Internationale Anmeldenummer: PCT/EP2013/003520
(87) Internationale Veröffentlichungsnummer: WO 2014/079574

(56) Entgegenhaltungen:
- WO-A1-93/21624
- WO-A1-2007/036327
- WO-A2-02/089714
- US-A- 5 841 507
- US-A1- 2005 036 109
- US-B1- 7 651 220

## Beschreibung

Die vorliegende Erfindung betrifft ein Verfahren zum Reduzieren der von einem Träger einer Brille, deren Gläser bereichsweise abdunkelbar sind, wahrnehmbaren Helligkeit mindestens eines Objekts nach dem Oberbegriff von Anspruch 1 sowie eine Blendschutzbrille nach dem Oberbegriff von Anspruch 14.

Die vorliegende Anmeldung beansprucht die Priorität der provisorischen Patentanmeldung US 61/729,396 vom 22.11.2012 und der Patentanmeldung DE 10 2012 022 914.3 vom 23.11.2012, die durch Bezugnahme in die vorliegende Anmeldung aufgenommen werden.

Brillen, deren Gläser bereichsweise abdunkelbar sind, sind bekannt. Derartige Brillen werden im Folgenden als Blendschutzbrillen bezeichnet. Eine Blendschutzbrille ist nicht ganzflächig und gleichmäßig dunkel wie eine herkömmliche Sonnenbrille, sondern sie verdunkelt einzelne Bereiche, insbesondere solche Bereiche, aus denen mehr Licht ins Auge fällt als erwünscht ist, während sie Bereiche, aus denen wenig Licht kommt, heller lässt. Dies hat den Vorteil, dass sowohl helle als auch dunkle Partien des Gesichtsfeldes mit höherer Detailtreue bei geringerer Ermüdung wahrgenommen werden können. Durch die selektive Abdunklung sind die zuvor zu hellen, jetzt abgedunkelten Partien klarer, detailreicher und farbiger wahrnehmbar, weil sich das Auge an diesen Stellen an weniger Licht anpassen muss. Außerdem kann sich ein Wechsel zwischen Aufsetzen und Abnehmen der Sonnenbrille in vielen Fällen erübrigen.

Aus US 5,841,507 ist eine aktive Sonnenbrille zum selektiven Reduzieren der Lichtintensität in einem Gesichtsfeld eines Auges eines Benutzers bekannt. Hierfür wird das Gesichtsfeld des Benutzers von einem Sensor erfasst, der entsprechende Lichtintensitätssignale erzeugt. Die Lichtintensitätssignale werden unter Berücksichtigung vorgegebener Schwellwerte und einer benutzerabhängigen Anpassung verarbeitet. Wenn die Lichtintensitätssignale einen der Schwellwerte überschreiten, werden ein oder mehrere Elemente von Matrizen aus ansteuerbaren LCD-Elementen in den Gläsern der aktiven Sonnenbrille abgedunkelt. Mit Hilfe eines Pupillenpositionssensors kann eine Position der Pupille ermittelt werden.

In US 7,651,220 B1 ist ein System zum Abblocken von Blendpunkten offenbart, wobei ein Blendschutz oder die Gläser einer Schutzbrille bereichsweise abgedunkelt werden können. Durch eine Kalibration kann die Position eines Auges eines Benutzers zum Blendschutz bzw. zur Schutzbrille ermittelt werden.

Gemäß WO 02/089 714 A2 umfasst eine Blendschutzvorrichtung ein elektrooptisches Element mit einer transparenten Fläche, die eine Vielzahl elektrisch adressierbarer Pixel enthält, durch welche ein Gesichtsfeld eines Sensors bzw. eines Auges beobachtet werden kann. Mit einer Bilderfassungseinrichtung wird ein Bild einer Beleuchtungsquelle in dem Gesichtsfeld erfasst. Ein Prozessor wertet das Bild aus, um die Pixel zu ermitteln, deren Transmission zur Reduzierung der wahrgenommenen Helligkeit der Lichtquelle zu verändern ist. Die entsprechenden Pixel des elektrooptischen Elements werden zur Reduzierung der Helligkeit der Lichtquelle angesteuert. Die Vorrichtung umfasst ein Subsystem zur Erfassung der Position der Pupillen eines Benutzers.

Aus WO 93/21624 A1 ist ein System zum Reduzieren der Intensität von Lichtstrahlen bekannt, das ein elektro-optisches Element mit einer optisch transparenten Oberfläche mit elektrisch adressierbaren Pixeln umfasst. Gemäß US 2005/036109 A1 wird für eine verbesserte Aberrationskorrektur eines Linsensystems ein Pupillendurchmesser erfasst. Aus WO 2007/036327 A1 ist ein Verfahren zum gesteuerten Einstellen einer Brechkraft eines Brillenglases einer Brille bekannt, wobei ein Durchmesser der Pupille mindestens eines Auges eines Benutzers ermittelt wird.

Blendschutzvorrichtungen, die in einem großen Abstand zu den Augen eines Benutzers angeordnet sind, sind aus prinzipiellen Gründen zur selektiven Abdunklung von Blendquellen unzureichend. Bekannte Blendschutzbrillen sind hinsichtlich der Abdunklung von Blendquellen und der Wahrnehmbarkeit von Bereichen außerhalb der Blendquellen ebenfalls nicht optimal; beispielsweise entsteht häufig um eine abgedunkelte Blendquelle herum ein dunklerer Bereich, der die Wahrnehmung von in diesem Bereich befindlichen Objekten beeinträchtigt.

Es ist Aufgabe der vorliegenden Erfindung, ein Verfahren zum Reduzieren der von einem Träger einer Brille, deren Gläser bereichsweise abdunkelbar sind, wahrnehmbaren Helligkeit mindestens eines Objekts anzugeben, wobei die erwähnten Nachteile möglichst vermieden werden. Weiterhin ist es Aufgabe der Erfindung, eine entsprechende Blendschutzbrille anzugeben.

Diese Aufgabe wird durch ein Verfahren gemäß Anspruch 1 sowie durch eine Blendschutzbrille gemäß Anspruch 14 gelöst.

Erfindungsgemäß wird ein Verfahren angegeben zum Reduzieren der von einer Person wahrnehmbaren Helligkeit mindestens eines Objekts, wobei die Person eine Brille trägt, deren Gläser bereichsweise abdunkelbar sind, d.h. eine Blendschutzbrille. Trägt eine Person, die im Folgenden als Träger bezeichnet wird, eine derartige Brille, so ist vor jedem Auge der Person ein Glas der Brille angeordnet, das bereichsweise abdunkelbar ist. Die die Brille tragende Person kann eine im Objektraum befindliche Szene durch die Gläser der Brille wahrnehmen. Das Verfahren bezieht sich auf die Helligkeitsreduzierung mindestens eines Objekts innerhalb des durch die Gläser der von der Person getragenen Brille bestimmten beobachtbaren Teils des Objektraums, der im Folgenden als Gesichtsfeld bezeichnet wird, insbesondere innerhalb der von dem Träger der Brille beobachteten Szene. Die Gläser der Blendschutzbrille können in an sich bekannter Weise ausgebildet sein und beispielsweise eine Matrix aus elektronisch ansteuerbaren elektrooptischen Elementen, insbesondere LCD-Elementen, die im Folgenden als Pixel bezeichnet werden, aufweisen, durch die pixelweise die Transmission des Brillenglases verändert werden kann. Durch Ansteuerung mehrerer, ggf. zusammenhängender, Pixel kann die Transmission in größeren Bereichen verändert werden.

Bei dem erfindungsgemäßen Verfahren wird ein Sehstrahl von mindestens einem Auge des Trägers der Brille zu dem mindestens einen Objekt bestimmt und hieraus ein Durchtrittspunkt, in dem der Sehstrahl durch das vor dem betreffenden Auge angeordnete Glas der Brille tritt, ermittelt. Der Durchtrittspunkt kann beispielsweise in einem Koordinatensystem bestimmt werden, das dem betreffenden Brillenglas zugeordnet ist, und liegt insbesondere in einer Ebene oder auch einer gekrümmten Fläche, die durch die Matrix der elektronisch ansteuerbaren Elemente gebildet wird. Zur Identifikation des Objekts und zur Ermittlung des Durchtrittspunkts kann eine Kamera, beispielsweise eine CCD-Kamera, an dem Gestell der Brille angeordnet sein, die die Szene erfasst, worin das Objekt enthalten ist; diese Kamera wird im Folgenden als Szenekamera bezeichnet. Das Objekt kann in einem von der Szenekamera aufgenommenen Bild aufgrund vorgebbarer Kriterien, die sich etwa auf die Helligkeit oder die Flächenhelligkeit des Objekts beziehen können, oder auch aufgrund von Daten, die von einem übergeordneten System empfangen werden, identifiziert werden. Aufgrund der Position des derart identifizierten Objekts kann der Durchtrittspunkt des Sehstrahls durch das vor dem mindestens einen Auge angeordnete Brillenglas ermittelt werden. Hierfür kann eine Steuerungseinrichtung vorgesehen und eingerichtet sein, um die von der Szenekamera erfassten Bilddaten zu empfangen, auszuwerten und den Durchtrittspunkt zu ermitteln. Es können auch mehrere Objekte und entsprechend mehrere Durchtrittspunkte ermittelt werden.

Das vor dem mindestens einen Auge angeordnete Brillenglas wird in einem Bereich um den ermittelten Durchtrittspunkt herum abgedunkelt; sofern mehrere Objekte und mehrere Durchtrittspunkte ermittelt worden sind, können entsprechend mehrere Bereiche abgedunkelt werden. Der mindestens eine abgedunkelte Bereich enthält den Durchtrittspunkt und ist beispielsweise als elliptische oder kreisförmige Fläche, wobei der Durchtrittspunkt der Mittelpunkt der Ellipse bzw. des Kreises ist, ausgebildet. Der abgedunkelte Bereich kann auch den überwiegenden Teil der Fläche eines Brillenglases oder das gesamte Brillenglas umfassen. Der abgedunkelte Bereich wird vorzugsweise gleichmäßig abgedunkelt, d.h. die Transmission des betreffenden Brillenglases ist im gesamten Bereich gleichmäßig gegenüber den nicht abgedunkelten Bereichen des Brillenglases reduziert. Als Maß für die Reduzierung der Transmission wird im Folgenden ein Abdunklungsgrad, der beispielsweise in Prozent des ohne Abdunklung bestehenden Transmissionsgrads angegeben werden kann, verwendet. Hierfür können insbesondere die innerhalb des abzudunkelnden Bereichs befindlichen elektrooptischen Elemente gleichmäßig abgedunkelt werden und hierfür von der Steuerungseinrichtung automatisch entsprechend angesteuert werden.

Erfindungsgemäß wird der aktuelle Durchmesser der Pupille des mindestens einen Auges ermittelt. Hierfür ist eine Pupillenerfassungseinrichtung vorgesehen, die beispielsweise eine CCD-Kamera umfassen kann, wobei durch Auswertung des von dieser aufgenommenen Bildes der Iris des Auges der Pupillendurchmesser bestimmt werden kann. Weiterhin ist erfindungsgemäß vorgesehen, dass die Größe und/oder der Abdunklungsgrad des abgedunkelten Bereichs von dem derart ermittelten Pupillendurchmesser abhängig ist bzw. sind. Insbesondere wird der ermittelte aktuelle Pupillendurchmesser des Trägers mit vorbestimmbaren Schwellwerten verglichen, die zwischen zuvor ermittelten, individuellen Werten für den Maximal- und den Minimaldurchmesser der Pupille liegen. Liegt der aktuelle Pupillendurchmesser unterhalb eines ersten Schwellwerts, kann darauf geschlossen werden, dass es dem Träger zu hell wurde, und somit wird eine Abdunklung eingeschaltet oder verstärkt; liegt der Pupillendurchmesser oberhalb eines zweiten Schwellwerts, kann darauf geschlossen werden, dass es dunkler wurde, und es wird eine aktivierte Abdunklung abgeschwächt oder ausgeschaltet. Der Pupillendurchmesser wird somit bei der Ansteuerung des Brillenglases zur Abdunklung eines Bereichs berücksichtigt. Die Steuerungseinrichtung kann hierfür zur Ansteuerung der Pupillenerfassungseinrichtung und zur Auswertung der von dieser gelieferten Daten zur Ermittlung des Pupillendurchmessers sowie zur Ansteuerung der elektrooptischen Elemente des Brillenglases entsprechend dem ermittelten Pupillendurchmesser ausgebildet sein. Insbesondere läuft das beschriebene Verfahren automatisch und kontinuierlich bzw. in einem Zeittakt ab, der durch die Reaktions- und Verarbeitungszeiten der beteiligten Systemkomponenten bestimmt wird.

Erfindungsgemäß ist erkannt worden, dass eine helligkeitsbedingte Reaktion einer Person, insbesondere der Durchmesser der Pupille, einen Rückschluss darauf zulässt, ob die Person durch eine Lichtquelle geblendet wird oder nicht, und ebenso, dass eine Reduzierung oder Ausschaltung der Blendung in verbessertem Masse gelingt, wenn die Gläser einer Blendschutzbrille in einer Weise angesteuert werden, die den Durchmesser der Pupille des Auges des Trägers der Brille berücksichtigt. Vom Pupillendurchmesser wird somit ein Abblendungsbedürfnis des Trägers abgeleitet. Ein Brillenglas hat einen so geringen Abstand von dem Auge, dass ein Punkt des Brillenglases und damit auch ein durch ein elektrooptisches Element gebildetes Pixel nicht vom Auge fokussiert werden kann. Andererseits können durch bereichsweise Abdunklung des Brillenglases Partien des wahrgenommenen Bildes gezielt beeinflusst werden. Dadurch, dass der Pupillendurchmesser für die Berechnung des abzudunkelnden Bereichs auf der Brille genutzt wird, kann ein abgedunkelter Bereich des Brillenglases so berechnet werden, dass dieser für das Auge mit der Umgebung verschmilzt, so dass er vom Träger kaum noch bewusst wahrgenommen wird und nur ein angenehm abgeblendeter Seheindruck entsteht.

Dabei ist es vorteilhaft, dass in beiden Gläsern der Brille Bereiche um jeweils mindestens einen Durchtrittspunkt abgedunkelt werden, wobei aufgrund der Geometrie der Brille und des Abstands der Augen aus dem für ein Brillenglas ermittelten Durchtrittspunkt ein entsprechender Durchtrittspunkt für das andere Brillenglas berechnet werden kann; eine entsprechende Zuordnung kann etwa bei einer Kalibrierungsmessung ermittelt werden. Da Objekte, deren Helligkeit reduziert werden soll, häufig in ausreichender Entfernung stehen, um den Abstand als unendlich anzunehmen, kann hierdurch in vielen Fällen eine ausreichende Reduzierung der Helligkeit erzielt werden. Eine weiter verbesserte Helligkeitsreduzierung kann gelingen, wenn das erfindungsgemäße Verfahren für beide Gläser der Brille durchgeführt wird, d.h. es wird ein jeweiliger Durchtrittspunkt eines Sehstrahls von beiden Augen zu dem mindestens einen Objekt durch das jeweilige Glas der Brille bestimmt und dieses in einem Bereich um den mindestens einen Durchtrittspunkt abgedunkelt. Ebenso ist es häufig ausreichend, den Pupillendurchmesser eines Auges zu ermitteln und hiermit die Größe bzw. den Abdunklungsgrad der abgedunkelten Bereiche in beiden Gläsern zu bestimmen. Es können aber auch die Pupillendurchmesser beider Augen ermittelt und jeweils zur Ansteuerung des betreffenden Glases genutzt werden. Wenn im Folgenden das Verfahren mit Bezug auf ein Auge beschrieben wird, so bezieht sich dies jeweils insbesondere auch auf beide Augen.

Vorzugsweise ist die Größe des mindestens einen abgedunkelten Bereichs derart von dem Pupillendurchmesser abhängig, dass der abgedunkelte Bereich umso größer gewählt wird, je größer der Pupillendurchmesser ist. Die Größe des abgedunkelten Bereichs kann von weiteren Einflussgrößen abhängen; ferner kann der gewählte Abdunklungsgrad vom Pupillendurchmesser abhängen. Bei einer Punktquelle wird insbesondere eine Kreisfläche um den Durchtrittspunkt abgedunkelt, wobei der Kreisdurchmesser geringer als der Pupillendurchmesser ist; bei einer schräg auf das Brillenglas scheinenden Punktquelle kann die Fläche entsprechend elliptisch geformt sein. Hierdurch kann eine weitgehende Helligkeitsreduzierung eines Objekts bei gleichzeitig nahezu unveränderter Helligkeit benachbarter Objekte erreicht werden.

Gemäß einer bevorzugten Ausführungsform der Erfindung ist der Grad der Abdunklung des abgedunkelten Bereichs von einer Empfindlichkeitsfunktion abhängig, wobei die Empfindlichkeitsfunktion personenunabhängig oder, in bevorzugter Weise, personenabhängig vorgegeben sein kann. Insbesondere kann die Empfindlichkeitsfunktion durch eine manuell oder automatisch durchgeführte Kalibrierung individuell anpassbar sein. Die Empfindlichkeitsfunktion stellt eine Beziehung dar zwischen dem ermittelten Pupillendurchmesser und dem Abdunklungsgrad einer abgedunkelten Fläche des Brillenglases, durch die die Helligkeit des Objekts reduziert wird. Auf den Pupillendurchmesser nimmt wiederum insbesondere eine wahrgenommene Helligkeit Einfluss. Ferner kann der Abdunklungsgrad in vorteilhafter Weise auch direkt von einer Helligkeit im Gesichtsfeld des Trägers der Brille abhängen. Die Helligkeit kann im einfachsten Fall von einem nach vorne gerichteten Helligkeitssensor gemessen werden, kann aber auch als durchschnittlicher Helligkeitswert aus einem von der Szenekamera aufgenommenen Bild ermittelt werden. Eine weitere Verbesserungsmöglichkeit ist, die Helligkeitswerte aus einem Bereich des Bilds der Szenekamera zu ermitteln, der nahe dem Bereich liegt, den der Träger betrachtet; für den letztgenannten Fall ist eine automatische Erkennung der Blickrichtung, etwa durch Erfassung der Pupillenposition, vorteilhaft. Bei der Bestimmung des Abdunklungsgrads kann es ferner vorgesehen sein, dass bei einer Helligkeit, die geringer ist als eine - ggf. von weiteren Einflussgrößen oder der Zeit abhängige - Minimalhelligkeit keine Abdunklung erfolgt, etwa weil diese Helligkeit zu gering ist bzw. die Objekte in der Szene zu lichtschwach sind, um eine Blendwirkung auszulösen. Auch bei einer gleichmäßigen ganzflächigen Abdunklung kann der Abdunklungsgrad gemäß einer Empfindlichkeitsfunktion vom Pupillendurchmesser abhängen. Hierdurch ist es möglich, individuell und/oder im Verlauf der Zeit unterschiedliche Bedürfnisse des Trägers der Brille zu berücksichtigen. Im wachen, gesunden Zustand kann beispielsweise eine Empfindlichkeit für eine Blendung durch eine Lichtquelle weniger ausgeprägt sein als im Zustand von Müdigkeit, Krankheit oder fortschreitendem Alter. Des Weiteren kann hierdurch auch die Adaptionszeit des Auges an plötzliche Helligkeitsänderungen berücksichtigt werden.

Weiterhin ist es bevorzugt, dass die Größe und die Form des abgedunkelten Bereichs von der Größe und der Form des Objekts abhängen. In besonders vorteilhafter Weise kann es vorgesehen sein, dass bei einem Objekt, das eine Punktquelle oder näherungsweise eine Punktquelle darstellt, der abgedunkelte Bereich kreisförmig ausgebildet ist, wobei die Größe der abgedunkelten Kreisfläche von der ermittelten Größe der Pupille des betreffenden Auges abhängt. Bei einem flächigen Objekt kann der abgedunkelte Bereich eine entsprechende Form haben und in seiner Größe etwa dem Objekt entsprechen. Dabei wird der abgedunkelte Bereich vorzugsweise mit einem in allen Pixeln gleichen Abdunklungsgrad abgedunkelt.

Gemäß einer bevorzugten Ausführungsform der Erfindung wird aus einer erfassten zeitlichen Veränderung des Pupillendurchmessers darauf geschlossen, ob eine Blendwirkung vorliegt. Hierfür wird die zeitliche Veränderung des Pupillendurchmessers erfasst und beispielsweise nur dann, wenn der Pupillendurchmesser innerhalb eines vorgebbaren Zeitintervalls um mehr als einen vorgebbaren Kontraktionsschwellwert abnimmt, die Abdunklung eines entsprechenden Bereichs veranlasst. Gemäß diesem Aspekt der Erfindung ist erkannt worden, dass die Tatsache, ob eine Lichtquelle als Blendquelle empfunden wird, an einer Kontraktion der Pupille erkannt werden kann. Der Abdunklungsgrad des abgedunkelten Bereichs hängt somit in der Weise vom Pupillendurchmesser ab, dass nur dann überhaupt eine Abdunklung erfolgt, wenn die zeitliche Veränderung des Pupillendurchmessers einen vorgebbaren Minimalwert überschreitet. Der Kontraktionsschwellwert kann je nach Person, Alter und/oder Situation unterschiedlich sein und kann bei einer Kalibrierung individuell erfasst und gespeichert werden. Auf diese Weise ist eine besonders sichere automatische Erkennung einer Blendwirkung möglich.

Alternativ oder zusätzlich kann in vorteilhafter Weise der Abdunklungsgrad des abgedunkelten Bereichs vom Betrag und der Richtung einer zeitlichen Veränderung des Pupillendurchmessers abhängig sein. Gemäß diesem Aspekt der Erfindung ist erkannt worden, dass sich die Pupille bei plötzlich eintretender Dunkelheit in einem vorgebbaren Zeitintervall nur höchstens um einen ersten Maximalbetrag vergrößern und bei plötzlich eintretender Helligkeit in einem vorgebbaren Zeitintervall nur höchstens um einen zweiten Maximalbetrag verkleinern kann. Entspricht eine erfasste zeitliche Veränderung des Pupillendurchmessers näherungsweise dem ersten oder dem zweiten Maximalbetrag innerhalb des vorgebbaren Zeitintervalls, so kann daher darauf geschlossen werden, dass die Pupille in Kürze ihren maximalen bzw. minimalen Durchmesser erreichen wird, und die Brille zur Abdunklung eines abzudunkelnden Bereichs entsprechend dem maximalen bzw. minimalen Durchmesser der Pupille angesteuert werden. Der erste und der zweite Maximalbetrag können je nach Person, Alter und/oder Situation unterschiedlich sein und können individuell erfasst und gespeichert werden. Auf diese Weise kann eine besonders komfortable Reduzierung einer Blendwirkung erreicht werden.

Weiterhin ist es bevorzugt, dass nur dann eine Abdunklung eines Bereichs erfolgt, wenn der ermittelte Pupillendurchmesser einen unteren Schwellwert, der als Anschaltschwelle bezeichnet wird, unterschreitet, und dass keine oder eine verringerte Abdunklung mehr erfolgt, wenn der Pupillendurchmesser einen oberen Schwellwert, der als Abschaltschwelle bezeichnet wird, überschreitet. Hierbei wird ausgenutzt, dass bei einer sehr kleinen Pupille angenommen werden kann, dass eine weitgehende Helligkeitsreduzierung bereits durch die Pupille erfolgt und zur weiteren Anpassung des Auges eine Adaption der Netzhaut wünschenswert ist. Die Anschalt- und die Abschaltschwelle können je nach Person, Alter und/oder Situation unterschiedlich sein und können individuell erfasst und gespeichert werden Hierdurch kann eine Vereinfachung und eine besonders sichere Durchführung des Verfahrens zur Vermeidung einer Blendwirkung erreicht werden. Ferner wird hierdurch eine Hysterese eingeführt, die den Komfort weiter erhöht.

Gemäß einer bevorzugten Ausführungsform der Erfindung wird eine Position der Pupille des mindestens einen Auges erfasst und der Durchtrittspunkt des Sehstrahls vom Mittelpunkt der Pupille zum Objekt durch das dem Auge zugeordnete Brillenglas ermittelt. Hierfür kann die Pupillenerfassungseinrichtung derart ausgebildet sein, dass nicht nur der Durchmesser der Pupille, sondern auch die Position der Pupille relativ zu dem vor dem betreffenden Auge angeordneten Glas der Brille ermittelt wird. Sofern das Objekt flächig ist und ein Mittelpunkt oder ein Helligkeitsschwerpunkt des Objekts ermittelt werden kann, ist der Durchtrittspunkt insbesondere durch den Sehstrahl vom Mittelpunkt der Pupille zum Mittelpunkt bzw. Helligkeitsschwerpunkt des Objekts bestimmt. Hierdurch kann eine genauere Positionierung des abgedunkelten Bereichs vor dem betreffenden Auge erreicht und die Blendwirkung noch effektiver reduziert werden.

Weiterhin ist es bevorzugt, dass eine Mimik des Trägers der Brille erfasst wird und die Größe und/oder der Grad der Abdunklung des abgedunkelten Bereichs von der Mimik abhängig ist. Ebenso kann im Fall einer gleichmäßigen ganzflächigen Abdunklung der Abdunklungsgrad von der Mimik abhängig sein. Zur Erfassung der Mimik kann eine Mimikerfassungseinrichtung vorgesehen sein, oder die Pupillenerfassungseinrichtung kann auch zur Erfassung der Mimik ausgebildet sein. Die Mimik äußert sich insbesondere in Bewegungen der Augenlider, der Wangen und/oder der Augenbrauen. Gemäß diesem Aspekt der Erfindung ist erkannt worden, dass auch die Mimik einen Rückschluss darauf zulässt, ob und ggf. in welchem Ausmaß eine Lichtquelle eine Blendwirkung verursacht. Auch die Interpretation der Mimik, d.h. der Zusammenhang zwischen einer erfassten Mimikreaktion des Trägers und einer Veränderung der Abdunklung, kann vorzugsweise durch eine Kalibrierung individuell angepasst werden. Dadurch, dass eine Mimik des Trägers der Brille erfasst wird, kann die Sicherheit bei der Erkennung einer Blendquelle und der Reduzierung der wahrnehmbaren Helligkeit der Blendquelle weiter erhöht werden. Die Erfassung der Mimik kann alternativ dazu verwendet werden, auch ohne Erfassung des Pupillendurchmessers auf eine Blendwirkung zu schließen und eine Abdunklung eines Bereichs des Brillenglases entsprechend anzusteuern.

Vorzugsweise wird mindestens ein Situationsparameter des Trägers der Brille erfasst, der beispielsweise anzeigt, ob der Träger der Brille ein Gespräch führt oder ob sein Gesicht einem starken Wind ausgesetzt ist. In beiden Fällen kann darauf geschlossen werden, dass eine Mimik, die in einer Situation ohne Gespräch bzw. ohne Wind als Hinweis auf eine Blendwirkung gelten würde, durch das Gespräch bzw. den Wind verursacht ist. Durch eine entsprechende Bewertung kann dann, ggf. in Abhängigkeit von weiteren Einflussgrößen, auf die Abdunklung eines entsprechenden Bereichs verzichtet werden.

Gemäß einer bevorzugten Ausführungsform der Erfindung wird der zeitliche Verlauf des Pupillendurchmessers erfasst und vor seiner Auswertung einer Tiefpassfilterung unterzogen. Hierdurch kann verhindert werden, dass rasche Änderungen des Pupillendurchmessers, wie sie normalerweise ständig erfolgen, eine Auswirkung auf die Abdunklung des entsprechenden Bereichs haben. Ebenso kann es in vorteilhafter Weise vorgesehen sein, dass ein Signal zur Ansteuerung der Gläser zur Abdunklung tiefpassgefiltert wird, um störende Reaktionen auf rasch wechselnde Helligkeiten eines Objekts zu vermeiden. Hierdurch können der Komfort und die Erkennbarkeit einer Szene weiter verbessert werden.

In bevorzugter Weise wird eine Bewegung des Objekts erfasst, die sich insbesondere in einer Bewegung des Durchtrittspunkts relativ zum Brillenglas zeigt. Das Glas wird sodann zu einer vorausschauenden Abdunklung angesteuert, so dass eine etwaige Zeitverzögerung durch die Erfassung und Verarbeitung der Daten durch die Steuerungseinrichtung sowie durch eine etwaige Reaktionszeit der Gläser zur Umsetzung der Abdunklung kompensiert wird. Hierdurch ist auch bei bewegten Szenen eine optimale Reduzierung der wahrgenommenen Helligkeit erreichbar.

Weiterhin ist es bevorzugt, dass durch Auswertung des erfassten Pupillendurchmessers eine Störung der Befindlichkeit des Trägers der Brille erkannt wird. Aufgrund einer derart erkannten Störung, etwa einer Krankheit oder des Nachlassens der Aufmerksamkeit, kann ein Signal oder eine Information an den Träger der Brille oder auch an eine übergeordnete Überwachungsinstanz ausgegeben werden. Hierdurch kann die Sicherheit insbesondere bei der Verwendung des erfindungsgemäßen Verfahrens beim Führen eines Kraftfahrzeugs erhöht werden.

Gemäß einer bevorzugten Ausführungsform der Erfindung ist es vorgesehen, dass eine Mehrzahl von Objekten in der Szene erkannt werden, wovon ein Objekt gegenüber den anderen Objekten hervorzuheben ist. Die vom Träger der Brille wahrnehmbare Helligkeit der nicht hervorzuhebenden Objekte wird dadurch reduziert, dass das Brillenglas in Bereichen um Durchtrittspunkte von Sehstrahlen von einem Auge zu den nicht hervorzuhebenden Objekten abgedunkelt wird. In diesem Fall erfolgt in einem Bereich des Glases, der dem Sehstrahl zu dem hervorzuhebenden Objekt entspricht, keine Abdunklung, während außerhalb dieses Bereichs eine Abdunklung erfolgt. Hierdurch wird das hervorzuhebende Objekt für den Träger einfacher erkennbar. Das hervorzuhebende Objekt kann dabei insbesondere aufgrund von außen übermittelter Daten identifiziert werden, während die übrige Szene als nicht hervorzuhebendes Objekt angesehen wird. Hierdurch wird das betreffende Objekt herausgehoben und subjektiv aufgehellt. Auf diese Weise ist eine verbesserte Erkennung des durch die von außen übermittelten Daten identifizierten Objekts möglich. Das hervorzuhebende Objekt kann beispielsweise ein von einem Navigationssystem empfohlener Weg sein. Die Daten, die das hervorzuhebende Objekt angeben, können von dem Navigationssystem oder auch beispielsweise im Rahmen eines Augmented-Reality-Systems (Erweiterte Realität) übermittelt werden. Alternativ oder zusätzlich kann es vorgesehen sein, dass ein vorbestimmbarer Bereich mindestens eines Glases der Brille, in den nach Art einer Datenbrille oder im Rahmen eines Augmented-Reality-Systems eine Einblendung von Bildern, Texten oder Zeichen erfolgt, abgedunkelt wird, um die Erkennbarkeit der Einblendung zu verbessern. Der Abdunklungsgrad kann dabei von der Helligkeit des hierdurch abgedeckten Bereichs der Szene abhängen. Sowohl die Hervorhebung eines Objekts als auch die Kontrasterhöhung eingeblendeter Bilder, Text oder Zeichen kann auch ohne die Erfassung eines Pupillendurchmessers vorteilhaft sein.

Vorzugsweise ist es vorgesehen, dass sowohl eine Abdunklung eines oder mehrerer lokalisierter Bereiche des Brillenglases zur Helligkeitsreduzierung einzelner punktförmiger oder flächiger Blendquellen, eine Abdunklung eines überwiegenden Teils des Glases zur Hervorhebung eines Objekts oder auch eine ganzflächige Abdunklung nach Art einer normalen Sonnenbrille erfolgen kann. Alternativ oder zusätzlich zur Vorgabe durch ein externes System kann es in vorteilhafter Weise vom ermittelten Kontrastumfang innerhalb eines Bilds der Szenekamera abhängig sein, welche Art der Abdunklung gewählt wird. So kann etwa bei geringem Kontrastumfang die ganzflächige Abdunklung gewählt werden. Bei kleinen, sehr hellen Quellen wird eine an Punktquellen angepasste Abdunklung angesteuert, wobei die Fläche der abdunkelnden Scheibe vom Pupillendurchmesser abhängig ist. Bei Flächen, deren Helligkeit weit über der der Umgebung liegt, kann die Flächenabdunklung gewählt werden, wobei abhängig vom Pupillendurchmesser ein unscharfer Übergangsbereich optimal in das wahrgenommene Bild gelegt werden kann, insbesondere so, dass er situationsabhängig innerhalb, außerhalb oder über den Rand der abzudunkelnden Fläche verläuft.

Eine erfindungsgemäße Blendschutzbrille umfasst zwei elektronisch ansteuerbare, bereichsweise abdunkelbare Gläser, ein Gestell zum Halten der Gläser vor den Augen eines Trägers, eine elektronische Kamera zum Erfassen einer von dem Träger beobachtbaren Szene, eine Pupillenerfassungseinrichtung und eine Steuerungseinrichtung, wobei die Steuerungseinrichtung zum Ermitteln eines Durchtrittspunkts eines Sehstrahls von mindestens einem Auge des Trägers zu einem von der Szenekamera erfassten Objekt durch das vor dem Auge angeordnete Glas der Blendschutzbrille und zum Ansteuern der Gläser zum Abdunkeln eines Bereichs um den Durchtrittspunkt ausgebildet ist. Erfindungsgemäß ist die Pupillenerfassungseinrichtung zum Erfassen eines aktuellen Pupillendurchmessers des mindestens einen Auges des Trägers ausgebildet und die Steuerungseinrichtung zum Ansteuern der Gläser zum Abdunkeln des Bereichs des Glases in Abhängigkeit vom aktuellen Pupillendurchmesser eingerichtet. Insbesondere kann es vom aktuellen Pupillendurchmesser abhängig sein, ob überhaupt eine Abdunklung eines Bereichs erfolgt, und sofern eine Abdunklung erfolgt, ist die Größe und/oder der Abdunklungsgrad des Bereichs vom Pupillendurchmesser abhängig. In vorteilhafter Weise kann die Steuerungseinrichtung am Gestell der Blendschutzbrille angeordnet oder in dieses integriert sein, kann aber auch als separates Gerät ausgeführt und mit dem Gestell über eine Leitung verbunden sein. Ebenso kann eine Stromversorgung der Blendschutzbrille am Gestell angeordnet oder separat sein.

Gemäß einer bevorzugten Ausführungsform ist die Blendschutzbrille, insbesondere die Steuerungseinrichtung der Blendschutzbrille, über Nahfunk wie beispielsweise Bluetooth und/oder eine Sprachsteuerung bedienbar. Alternativ oder zusätzlich können entsprechend klein ausgebildete Schalter bzw. Taster an der Brille angeordnet sein. Hierdurch wird eine besonders einfache Bedienung ermöglicht.

In vorteilhafter Weise kann die Blendschutzbrille derart ausgebildet sein, dass sie eine Mehrzahl von Betriebsweisen aufweist, wobei mindestens eine Betriebsweise mit verringerter Stromaufnahme vorgesehen ist. Insbesondere kann die Steuerungseinrichtung eine Mehrzahl von Verarbeitungsmodi aufweisen, wobei mindestens ein Modus eine geringere Stromaufnahme erfordert. In diesem Modus kann beispielsweise eine Datenerfassung mit verringerter Frequenz erfolgen und/oder die Berechnung der abzudunkelnden Bereiche kann in vereinfachter Weise erfolgen. Hierdurch kann die Stromaufnahme der Blendschutzbrille verringert werden.

Vorzugsweise ist die Blendschutzbrille zur Durchführung des zuvor beschriebenen erfindungsgemäßen Verfahrens ausgebildet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung. Es zeigen:
Fig. 1 ein Ausführungsbeispiel einer erfindungsgemäßen Blendschutzbrille;
Fig. 2 ein Beispiel einer Szene mit blendender Sonne ohne Blendschutz, mit einer gewöhnlichen Sonnenbrille, mit einer bekannten Blendschutzbrille sowie mit einer erfindungsgemäßen Blendschutzbrille;
Fig. 3A bis B'" zwei Ausführungsformen eines erfindungsgemäßen Verfahrens zum Abdunkeln einer punktförmigen, blendenden Lichtquelle;
Fig. 4A bis C eine blendende Fläche sowie zwei Ausführungsformen eines erfindungsgemäßen Verfahrens, um die blendende Fläche abzudunkeln;
Fig. 5 ein Beispiel zur Ermittlung einer Einschalt- und einer Abschaltschwelle der Abdunklung anhand des Pupillendurchmessers;
Fig. 6 ein weiteres Beispiel zur Ermittlung der Einschalt- und der Abschaltschwelle der Abdunklung anhand des Pupillendurchmessers;
Fig. 7 Formen von Veränderungen der Augenlider, die erfindungsgemäß erfasst und ausgewertet werden können;
Fig. 8 Formen von Veränderungen der Augenbrauen, die erfindungsgemäß erfasst und ausgewertet werden können;
Fig. 9 Formen von Veränderungen der Wangen in Augennähe, die erfindungsgemäß erfasst und ausgewertet werden können;
Fig. 10 ein Auge mit verschiedenen Blickrichtungen aus Sicht einer seitlich am Brillengestell angebrachten Kamera;
Fig. 11 einen Querschnitt eines Auges mit seitlich angebrachter Pupillenerfassungskamera;
Fig. 12 das Verfahren gemäß Fig. 5 bei einer zuschaltbaren Sonnenbrille;
Fig. 13 das Verfahren gemäß Fig. 6 bei einer zuschaltbaren Sonnenbrille;
Fig. 14 ein Beispiel für eine durch eine Blendschutzbrille gemäß einem Ausführungsbeispiel mit einer Augmented-Reality-Funktion beobachtete Szene;
Fig. 15 ein Beispiel für eine durch eine Blendschutzbrille gemäß einem Ausführungsbeispiel mit einer Dateneinblendung beobachtete Szene.

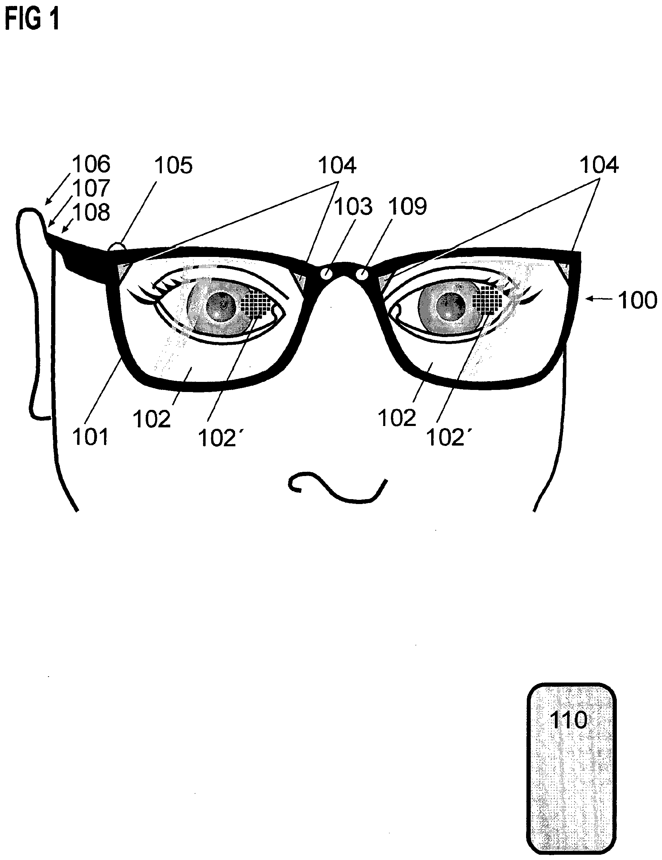

Wie in Fig. 1 dargestellt, umfasst eine erfindungsgemäße Blendschutzbrille 100 gemäß einem Ausführungsbeispiel folgende, teilweise optionale, Komponenten: Ein Brillengestell 101, zwei Brillengläser 102, die in beliebigen Bereichen abgedunkelt werden können, eine nach vorne gerichtete elektronische Szenekamera 103, eine Pupillenerfassungseinrichtung 104 für jedes Auge, einen Windsensor 105, einen Beschleunigungssensor 106, eine Steuerungseinrichtung 107, eine Stromversorgung 108, einen Helligkeitssensor 109 und ein externes Gerät 110 zur Bedienung bzw. Parametrierung der Blendschutzbrille 100.

Die Blendschutzbrille 100 umfasst, wie jede andere Brille, ein Brillengestell 101. Dieses wird hier auch als Träger für weitere Komponenten sowie für deren Verkabelung benötigt.

Die Brillengläser 102 müssen rasterartig an beliebigen Stellen abgedunkelt werden können. Hier können Verfahren eingesetzt werden, wie sie bei Displays und Monitoren bekannt sind, wie etwa bei einem LCD-Display. Dabei ist es vorteilhaft, graduell abdunkeln zu können. Eine graduelle Abdunklung ist ersetzbar durch eine Rasterung bei feiner Pixelauflösung. In Fig. 1 sind mit dem Bezugszeichen 102' beispielhaft Flächen bezeichnet, die rasterartig abgedunkelt sind.

Auf der Brille 100 ist eine Szenekamera 103 zur Erfassung der betrachteten Szene angebracht. Sie ist nach vorn gerichtet und sollte mindestens denselben Bereich des Objektraums abdecken, der von den Augen durch die Brillengläser 102 beobachtet werden kann. Zusätzlich kann ein ebenso nach vorn gerichteter Helligkeitssensor 109 vorgesehen sein.

Die Szenekamera 103 kann so ausgelegt werden, dass sie den UV- und IR-Bereich nicht erfasst, um Fehler bei der Abdunklung zu verhindern. Als Ausgleich sollte dann ein UV- und IR-Filter in die Gläser 102 eingearbeitet sein, um die Augen vor Strahlung schützen, die von der aktiven Abdunklung der Brille - die von den Bildern der Szenekamera 103 abhängt - nicht gefiltert wird. Für die Bildverarbeitung ist lediglich ein Graustufenbild erforderlich. Eine Farbkamera ist somit nicht notwendig. Mit einer Graustufenkamera sind eine höhere Lichtempfindlichkeit und eine kleinere Baugröße erreichbar. Die Szenekamera 103 kann auf dem Steg zwischen den beiden Gläsern 102 montiert sein.

Zur Pupillenerfassung können Techniken eingesetzt werden, die aus der Blickerfassung (Eye Tracking) bekannt sind. Ziel ist hier jedoch, abgesehen von der unten beschriebenen Ausführungsform, nicht die Erfassung des Punktes, auf den das Auge gerichtet ist, sondern der Durchmesser der Pupille, sowie ihre Position im Verhältnis zu den Gläsern 102. Die Pupillenerfassungseinrichtung 104 umfasst mindestens eine Kamera für jedes Auge (im Folgenden auch Augenkamera genannt), mindestens eine IR-Lichtquelle für jedes Auge, sowie eine Elektronik, die aus den aufgenommenen Bildern die Position und den Durchmesser der Pupillen ermittelt. Diese Elektronik schaltet die IR-Lichtquellen bei Bedarf zu. Die Augenkameras müssen dann natürlich im Frequenzbereich dieser Lichtquelle empfindlich sein. Die Elektronik kann auch Teil der Steuerungseinrichtung 107 sein. Mit Hilfe von Polarisationsfiltern vor den Kameras der Pupillenerfassungseinrichtung 104 können störende Spiegelungen auf den Augen reduziert werden.

Die Augenkameras sowie mögliche IR-Lichtquellen zur Pupillenvermessung könnten beispielsweise in unauffälliger Weise nahe dem Steg zwischen den Gläsern angebracht sein. Am günstigsten ist eine Position möglichst nah an der Sehachse. Bei den normalen Formen von Brillengläsern wäre das über den Augen. Dann ist allerdings eine im oberen Bereich entsprechend breite Fassung nötig, sofern die Augenkamera darin versteckt werden soll. Sofern dies für eine ausreichende Erfassung der Augenbewegungen notwendig ist, können weitere Augenkameras beispielsweise an der Außenseite der Brille angeordnet sein.

Bilder der Kameras der Pupillenerfassungseinrichtung 104 können auch zusätzlich dazu verwendet werden, beispielsweise ein Zusammenkneifen der Augen und/oder eine weitere Mimik des Trägers der Brille 100 zu erkennen. Es ist allerdings auch möglich, getrennte Kameras für die Pupillenvermessung und für die Mimikerkennung einzusetzen.

Die Blendschutzbrille 100 kann auch andere Funktionen aufweisen, etwa die Funktionalität einer Datenbrille haben. In diesem Fall, sowie bei Kombination mit anderen Anwendungen, können sowohl die Szenekamera 103 als auch die Augenkameras der Pupillenerfassungseinrichtung 104 für die weiteren Funktionen bzw. Anwendungen verwendet werden.

Eine Unterscheidung, ob Wind die Augen zum Zusammenkneifen veranlasst oder zu große Helligkeit, kann bei der Interpretation der Mimik helfen (siehe unten). Hierfür kann ein Windsensor 105 vorgesehen sein. Als Windsensor 105 kann auch ein Mikrofon zur Erfassung von Windgeräuschen verwendet werden. Ein solches Mikrofon kann auch zur Erkennung von Gesprächssituationen verwendet werden. Es ist auch möglich, einen Windsensor 105 und ein Mikrofon zur Gesprächserkennung getrennt in die Blendschutzbrille einzubauen. Ein Mikrofon kann auch bei einer Kombination mit anderen Funktionalitäten bzw. Geräten, wie beispielsweise einer Datenbrille oder einem Telefon, verwendet werden.

Ein oder mehrere in die Brille 100 integrierte Beschleunigungssensoren 106 können helfen, Kopfbewegungen zu bestimmen. Mit dieser Information können Verzögerungseffekte nach Kopfbewegungen ausgeglichen werden (siehe unten).

Die Steuerungseinrichtung 107 umfasst eine Steuerelektronik, die die Brillengläser zur Abdunklung von Bereichen ansteuert. Die Steuerungseinrichtung 107 kann in die Brille 100 integriert sein. Auch eine vollständige oder teilweise Auslagerung in ein externes Gerät 110 ist möglich, das dann über Kabel oder kabellos angeschlossen wird.

Für die Entscheidung, ob und welche Bereiche mit welchem Abdunklungsgrad abgedunkelt werden sollen, wird von der Steuerelektronik das Bild der Szenekamera 103 verwendet. Ferner berechnet die Steuerelektronik den Durchmesser sowie die Position der Pupillen aus den Daten der Pupillenerfassungseinrichtung, d.h. aus den Bildern der Augenkameras. Mit trigonometrischen Berechnungen ermittelt die Steuerelektronik die Flächen 102' auf den Gläsern 102, die abgedunkelt werden sollen. Basis dieser Berechnungen sind die als abzudunkelnd identifizierten Objekte der beobachteten Szene, die Positionen und Größen der Pupillen, die Positionen und Formen der Gläser 102, sowie die räumlichen Verhältnissen zwischen Augenkameras, Pupillen und Gläsern 102. Die Steuerungseinrichtung enthält auch eine Speichermöglichkeit für Daten. Dort können vom Brillenhersteller typische Adaptionskurven abgelegt sein. Aber auch vom Träger gemessene Pupillen- und Mimikreaktionen können gespeichert werden, um eine automatische Adaption an die individuellen Bedürfnisse zu erreichen. Die Steuerungselektronik kann weitere Funktionen aufweisen (siehe unten).

Die Stromversorgung 108 umfasst einen Energiespeicher, beispielsweise in Form von Batterien oder Akkus, und sollte möglichst in die Brille 100 integriert sein. Aus kosmetischen Gründen und aus Gründen der Gewichtsverteilung könnte ein guter Ort in den Brillenbügeln hinter den Ohren sein. Auch eine Unterbringung als externes Gerät ist möglich, das dann über Kabel angeschlossen werden muss.

Die Blendschutzbrille sollte möglichst kompakt gebaut sein. Die Möglichkeiten, Schalter zur Einstellung ihrer Funktionen unterzubringen sind daher begrenzt. Daher könnten notwendige Einstellungen beispielsweise auf einem externen Gerät 110 (beispielsweise einem Smartphone oder Tablet-PC) vorgenommen werden. Das externe Gerät 110 kommuniziert mit der Brille 100 über Nahfunk, beispielsweise Bluetooth. Vorzugsweise erfolgen Einstellungen und Anpassungen automatisch, so dass es für den Träger nicht oder nur selten notwendig ist, Einstellungen zu verändern.

Allerdings können zur Anpassung an einen Träger der Brille vorherige Einstellungen hilfreich sein. Einzustellende Parameter, die etwa von einem Optiker aufgenommen und eingestellt werden können, sind beispielsweise Geometriedaten wie der Augenabstand, die Entfernung und die Lage der Augen hinter den Gläsern sowie Angaben zur Geometrie der Augäpfel wie die Vorderkammertiefe und die Hornhautkrümmung. Weitere Daten betreffen die individuelle Adaptionsfähigkeit für Helligkeitsänderungen, insbesondere der mit einem Pupillometer messbare maximale und minimale Pupillendurchmesser, sowie einige Pupillendurchmesser bei vollständiger Adaption an unterschiedliche Helligkeiten, um daraus eine individuelle Kurve des Pupillendurchmessers in Abhängigkeit von der Helligkeit zu ermitteln. Besonders bei den Werten zum Pupillendurchmesser sind Abhängigkeiten vom Lebensalter, Kurz- und Weitsichtigkeit bekannt. Deshalb können zur Vereinfachung der Einstellung Werte für Klassen von Personen gespeichert sein, die in einfacher Weise auswählbar sind.

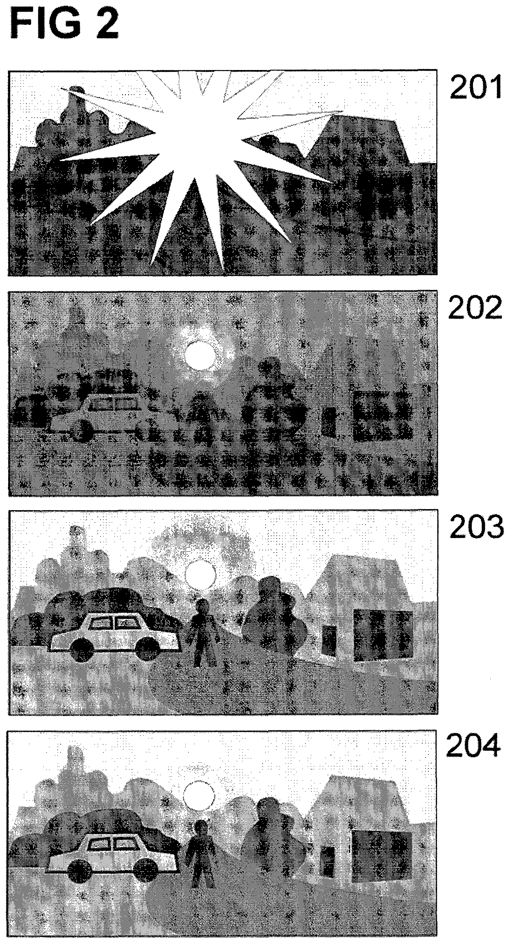

Zur Illustration des erfindungsgemäßen Verfahren ist in Fig. 2 eine Szene mit blendender Sonne gemäß dem subjektiven Eindruck bei Beobachtung in 4 Varianten dargestellt:
Das Bild 201 zeigt den Eindruck, den ein Beobachter ohne Blendschutz hat. Bei 202 ist der Eindruck mit einer gewöhnlichen Sonnenbrille, die einen gleichmäßigen Abdunklungsgrad aufweist, gezeigt. Das Bild 203 zeigt den Eindruck mit einer bekannten Blendschutzbrille, wobei ein dunklerer Ring um die abgedunkelte Sonne verbleibt, der die Erkennbarkeit der Szene in diesem Bereich erschwert. Die Breite des dunklen Rings hängt insbesondere vom Pupillendurchmesser ab. Mit dem erfindungsgemäßen Verfahren bzw. einer erfindungsgemäßen Blendschutzbrille ist dieser dunkle Ring deutlich reduziert (204).

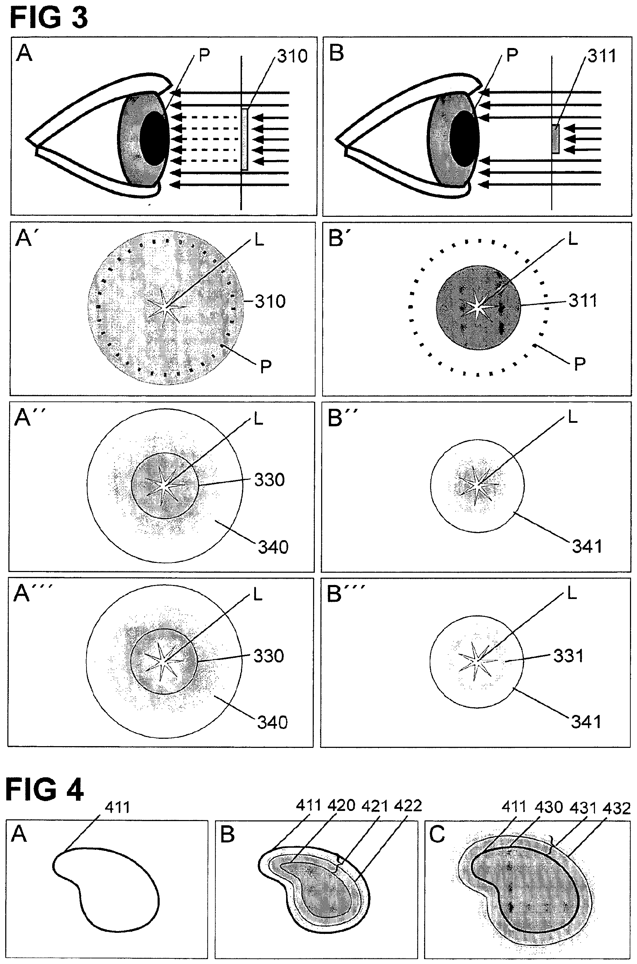

Zwei Beispiele für erfindungsgemäße Verfahren zur Reduzierung der Helligkeit einer punktförmigen, blendenden Lichtquelle sind in Fig. 3 gezeigt. Dabei kann mit einer großen, mäßig abgedunkelten kreisförmigen Fläche auf der Brille oder mit einer kleinen, stark abgedunkelten, kreisförmigen Fläche die Helligkeitsreduzierung erreicht werden:
Um eine punktförmige, blendende Lichtquelle, deren Licht in den Teilabbildungen A und B durch die Pfeile angedeutet ist, beispielsweise um 50% abzudunkeln, kann diese gemäß einem ersten Verfahren von einer kreisförmigen Fläche 310 abgedeckt werden, die mindestens die Größe der Pupille P hat und einen Abdunklungsgrad von 50% hat (s. Abbildung A). Entsprechend abgeschwächtes Licht der Lichtquelle erreicht somit die Pupille auf ihrer ganzen Fläche. Alternativ kann die blendende Lichtquelle von einer kreisförmigen Fläche 311 abgedeckt werden, die die halbe Fläche der Pupille P hat und 100% des Lichts filtert. Daher erreicht kein Licht der Lichtquelle aus dem Bereich, der von der Fläche 311 abgedeckt ist, die Pupille P. Allerdings erreicht Licht aus der ringförmigen Fläche, die über 311 hinausgeht, aber noch innerhalb der Pupillenfläche P liegt, die Pupille P in voller Stärke.

Wie in der Teilabbildung A' in einer zentralperspektivischen Darstellung gezeigt ist, wird die blendende Lichtquelle L gemäß dem ersten Verfahren von der kreisförmigen Fläche 310 abgedeckt, die mindestens die Größe der Pupille P hat und 50% des Lichts filtert. Gemäß Abbildung B' wird beim zweiten Verfahren die blendende Lichtquelle L wird von der kreisförmigen Fläche 311 abgedeckt, die die halbe Fläche der Pupille P hat und 100% des Lichts abblockt.

Die Teilabbildung A" der Fig. 3 zeigt die Abbildung auf der Netzhaut gemäß dem ersten Verfahren, wobei allerdings die Wirkung der Lichtquelle L noch nicht berücksichtigt ist. Somit entspricht diese Abbildung dem subjektiven Eindruck der abdunkelnden Fläche 310, wenn der Träger der Blendschutzbrille auf eine weiße Fläche sieht. Der Träger sieht eine Fläche 330, die die blendende Lichtquelle L, von der hier nur die Position markiert ist, und einen Teil ihrer Umgebung abdeckt. Die Fläche 340, die 330 umgibt, hat keinen scharfen Rand. Sie verläuft, bis sie nicht mehr wahrnehmbar ist, nach außen.

Die Teilabbildung B" zeigt die Abbildung auf der Netzhaut mit dem zweiten Verfahren, wobei ebenfalls die Wirkung der Lichtquelle L noch ignoriert ist. Somit entspricht diese Abbildung dem subjektiven Eindruck der abdunkelnden Fläche 311, wenn der Träger der Blendschutzbrille auf eine weiße Fläche sieht. Der Träger nimmt - im Gegensatz zu A" - kein großes, künstliches, durch die Brille erzeugtes Objekt wahr, sondern ein viel kleineres. Der in seinem Zentrum um 50% abgedunkelte Bereich verläuft schwächer werdend bis 341 nach außen.

Bild A"' der Fig. 3 zeigt den subjektiven Eindruck bzw. die wirkliche Abbildung auf der Netzhaut nach dem ersten Verfahren, einschließlich der Wirkung der Lichtquelle L. Der Träger der Blendschutzbrille sieht eine Fläche 330, die die blendende Lichtquelle L und einen Teil ihrer Umgebung abdeckt. Die Fläche 340, die 330 umgibt, hat keinen scharfen Rand. Sie verläuft, bis sie nicht mehr wahrnehmbar ist, nach außen. Die Lichtquelle L hat eine in ihre Umgebung ausstrahlende Wirkung. Wenn die Intensität der Abdunklung durch die Fläche 330 richtig ausgelegt ist, dunkelt sie das Zentrum der Lichtquelle auf ein angenehmes Niveau ab. Da die Fläche 330 jedoch weit in die Umgebung ragt, bleibt ein deutlich wahrnehmbarer, abdunkelnder Ring sichtbar. Was die Umgebung betrifft, ist die Abbildung dann korrekt, wenn eine gleichmäßig weiße Umgebung um die Lichtquelle L vorhanden ist, die nicht blendend hell ist.

In dem Bild B"' ist der subjektive Eindruck bzw. die Abbildung auf der Netzhaut nach dem zweiten Verfahren dargestellt, einschließlich der Wirkung der Lichtquelle L. Der Träger der Blendschutzbrille nimmt - im Gegensatz zu A" - kein großes, künstliches, durch die Brille erzeugtes Objekt wahr. Die Lichtquelle L ist klar erkennbar und um 50% ihrer Helligkeit reduziert. Der in seinem Zentrum um 50% abgedunkelte Bereich verläuft schwächer werdend bis 341 nach außen, wobei er von der ausstrahlenden Wirkung der Lichtquelle L überlagert ist. Es bleibt ein abdunkelnder Ring um die Lichtquelle L. Dieser Ring ist jedoch kleiner und wesentlich weniger intensiv als bei dem ersten Verfahren. Der Ring ist nicht völlig vermeidbar. Er ergibt sich einerseits aus der abdunkelnden Wirkung des unscharfen Übergangsbereichs der Fläche 331, dessen Breite vom Pupillendurchmesser und dem Abstand der Pupille von den Brillengläsern abhängt. Andererseits wird er vom Zentrum her durch die Strahlkraft der blendenden Lichtquelle aufgehoben. Die Breite des die Lichtquelle umgebenden "Strahlenkranzes" ergibt sich aus einer Reihe von Effekten, die von der Blendschutzbrille ebenfalls nicht beeinflussbar sind. Die unerwünschte Bildbeeinflussung ist bei dem zweiten Verfahren geringer als bei dem ersten. Insbesondere mit dem zweiten Verfahren lässt sich bei punktförmigen, blendenden Lichtquellen in vielen Fällen ein perfektes Ergebnis erzielen: Das Zentrum der Lichtquelle kann blendfrei wie mit einer Sonnenbrille betrachtet werden, während der unscharfe Übergangsbereich natürlich mit der Umgebung verschmilzt. Die Umgebung ist jedoch in voller Helligkeit sichtbar und nicht abgedunkelt wie bei einer normalen Sonnenbrille. Richtig berechnet ist die aktive Abdunklung auf diese Weise kaum noch bewusst wahrnehmbar und damit nicht mehr störend (s. Fig. 2, 204).

Auch wenn größere flächige Objekte abgedunkelt werden sollen, ist auf den Pupillendurchmesser zu achten. Auch hier gilt, dass die Breite des unscharfen Übergangsbereichs vom Pupillendurchmesser abhängt. Die abzudunkelnde Fläche auf der Brille kann jedoch, im Gegensatz zu punktförmigen Lichtquellen, nicht 100% dunkel gemacht werden, weil sonst Bildteile vollständig unsichtbar würden. Den Rand betreffend kann eine Strategie gewählt werden, die zwischen zwei Extremen liegt: (1.) den unscharfen Übergangsbereich innerhalb der abzudunkelnden Fläche auslaufen lassen oder (2.) ihn außerhalb der abzudunkelnden Fläche legen. Variante 1 hat Vorteile bei nicht allzu hellen Flächen, deren Rand klar wahrnehmbar bleibt. Variante 2 ist besser geeignet bei Flächen, die so hell sind, dass auch ihr Rand abgedunkelt werden muss, um einen angenehmen Seheindruck zu erzielen.

Die Steuerelektronik ist vorzugsweise so eingerichtet, dass bei einem geringem Helligkeitsunterschied zwischen einer blendenden Fläche und deren Umgebung die Variante 1 gewählt wird und bei extremem Helligkeitsunterschied die Variante 2. Bei einem Zwischenwert der Helligkeitsdifferenz kann die Steuerelektronik auch eine Strategie zwischen den beiden Varianten wählen und den unscharfen Übergangsbereich über die Flächengrenze verlaufen lassen.

In Fig. 4 ist schematisch eine blendende Fläche mit einem Rand 411 in der Szene dargestellt (A) sowie zwei Ausführungsformen eines erfindungsgemäßen Verfahrens, um die blendende Fläche abzudunkeln, nämlich (B) mit unscharfem Übergangsbereich innerhalb der blendenden Fläche und (C) mit unscharfem Übergangsbereich außerhalb der blendenden Fläche. Die Darstellungen B und C entsprechen in ihrer Vollständigkeit bzw. Unvollständigkeit den Teilabbildungen A" und B" in Fig. 3, d.h. die Leuchtkraft der blendenden Fläche ist nicht berücksichtigt.

Gemäß dem bei B dargestellten Verfahren erfolgt die Flächenabdunklung durch einen homogen abgedunkelten Bereich auf den Brillengläsern, dessen Rand mit dem Bezugszeichen 422 bezeichnet wird. Dieser Rand ist auf den Brillengläsern scharf begrenzt, jedoch vom Träger der Brille nicht erkennbar, da der Rand 422 auf der Netzhaut nicht fokussiert werden kann. Er geht nach außen und innen unmittelbar in den unscharfen Übergangsbereich 421 über, der sich etwa zu gleichen Teilen innerhalb und außerhalb des Rands 422 erstreckt. Der Übergangsbereich 421, wie auch die innere, homogen erscheinende Fläche mit dem Rand 420, liegen innerhalb der blendenden Fläche mit dem Rand 411. Am Rand 420 ist der vom Auge wahrgenommene Abdunklungsgrad des Übergangsbereichs 421 gleich dem des homogenen Bereichs und am Rand 411 der blendenden Fläche Null.

Wie bei C gezeigt, kann gemäß einer anderen Ausführungsform der Erfindung eine vollständige Flächenabdunklung erfolgen, so dass der Rand 430 der homogen abgedunkelt erscheinenden Fläche genau dem Rand 411 der blendenden Fläche entspricht. Ein unscharfer Übergangsbereich 431 ist am Rand 430 so dunkel wie die Fläche innerhalb von 430. Nach außen verschwindet er, d.h. der Abdunklungsgrad des Übergangsbereichs 431 wird außen Null. Der Übergangsbereich 431 erstreckt sich etwa zu gleichen Teilen innerhalb und außerhalb des Rands 432 des abgedunkelten Bereichs des Brillenglases.

Die Breiten der unscharfen Übergangsbereiche 421 und 431 sind gleich. Sie hängen von Pupillendurchmesser ab und können insbesondere gleich dem Pupillendurchmesser sein. In dem Fall, dass die blendende Fläche, die durch den Rand 411 begrenzt ist, wesentlich kleiner ist als die Breite des unscharfen Übergangsbereichs 421, 431, kann diese auch als Punktquelle angesehen werden und insbesondere das in den Figuren 3B bis B'" dargestellte Verfahren angewendet werden.

Um den Pupillendurchmesser zur individuellen und situationsabhängigen Anpassung der Abdunklung zu nutzen, kann in einem Speicher der Steuerungseinrichtung der Blendschutzbrille eine typische Adaptionskurve eines Menschen hinterlegt sein, beispielsweise in Abhängigkeit vom Alter des Trägers, die den typischen Pupillendurchmesser in Abhängigkeit von der Helligkeit kennt. Es ist dort der Pupillendurchmesser nach vollständiger Adaption an die jeweilige Helligkeit gespeichert. Zur Eingabe des Alters des Trägers kann beispielsweise ein externes Gerät, das mit der Blendschutzbrille über Nahfunk verbunden ist, genutzt werden.

Eine Abweichung des aktuellen Pupillendurchmessers, wie dieser von der Pupillenerfassungseinrichtung erfasst wird, von dem Durchmesser, der nach der Adaptionskurve bei der von der Szenekamera erfassten Helligkeit der beobachteten Szene zu erwarten ist, kann als Indiz für das Bedürfnis des Trägers nach stärkerer oder weniger starker Abdunklung verwendet werden. Die Steuerelektronik passt die Abdunklung, beispielsweise den Abdunklungsgrad oder eine Einschaltschwelle der Abdunklung, daraufhin kurzfristig entsprechend an. Die verzögerte Anpassung der Augen an Helligkeitsänderungen wird damit automatisch ausgeglichen. Außerdem passt sich so die Brille an geänderte Bedürfnisse des Trägers in Abhängigkeit von seiner Wachheit bzw. Ermüdung, sowie möglicher Erkrankungen an.

Durch Speicherung von mehreren von der Pupillenerfassungseinrichtung der Brille gemessenen Pupillendurchmessern in Abhängigkeit von der jeweils aktuellen Helligkeit können individuelle Adaptionskurven des Trägers erzeugt werden. Damit parametriert sich die Brille diesbezüglich selbst.

Das Auge hat zwei Mechanismen zur Anpassung an unterschiedliche Helligkeiten: Variation des Pupillendurchmessers und Anpassungsvorgänge in der Netzhaut. Die Anpassungen in der Netzhaut sind dabei die stärkeren, aber auch die langsameren. Schnelle Anpassungen erfolgen durch die Pupille, die in ca. einer halben Sekunde auf Helligkeitsänderungen reagiert. Die Netzhaut ist bei ihrer Adaption wesentlich langsamer. Für die Anpassung zwischen größter Helligkeit und größter Dunkelheit braucht sie 20 bis 30 Minuten, umgekehrt ca. 5 Minuten. Während dieser Adaptionszeit weicht die Pupille von ihrem durchschnittlichen Durchmesser ab, um die noch nicht vollendete Adaption der Netzhaut zu kompensieren. Einem Ausführungsbeispiel des Verfahrens liegt die vereinfachende Annahme zugrunde, dass der Pupillendurchmesser in einem weiten Helligkeitsbereich gleich groß ist, wenn die Netzhaut vollständig adaptiert ist. Erst im oberen und unteren Helligkeitsbereich weicht die Pupille dauerhaft von ihrem durchschnittlichen Durchmesser ab. Ausgehend von dieser Annahme kann die Blendschutzbrille die Phase der nötigen Adaptionsunterstützung der Pupille für die Netzhaut erkennen und unterstützen, indem sie den Pupillendurchmesser beobachtet. Daraus ergibt sich automatisch eine Funktionalität, die nicht nur eine Sonnenbrille für sonnige Tage im Freien ist, sondern auch eine Unterstützung des Auges für Übergangssituationen wie das Hinaustreten aus dunkleren Räumen ins Freie, selbst wenn es im Freien nicht so hell ist, dass bei längerem Aufenthalt eine Sonnenbrille nötig ist. Umgekehrt ergibt sich automatisch eine Funktionalität, bei der die Sonnenbrillenfunktion ab- oder zurückgeschaltet wird, wenn der Träger beispielsweise vom Sonnenlicht in einen Raum tritt.

Zur Steuerung der Abdunklung wird eine Helligkeit des von der Szenekamera aufgenommenen Bilds der beobachteten Szene ermittelt. Die Helligkeit soll dabei möglichst so ermittelt werden, wie es der physiologischen Wirkung, aufgrund derer das Auge den Pupillendurchmesser anpasst, entspricht. Das einfachste Berechnungsverfahren ist die Verwendung der Durchschnittshelligkeit des von der Szenekamera aufgenommenen Bildes. Verfeinerte Verfahren können beispielsweise Helligkeitsdifferenzen im Bild auswerten. Eine Möglichkeit dazu ist, sehr helle Bereiche bezüglich ihrer Intensität und/oder Fläche höher zu gewichten. Neben diesen Helligkeitsdifferenzen kann auch die Verteilung der Helligkeit bezüglich der Blickrichtung in die Berechnung des verwendeten Werts der Gesamthelligkeit einfließen, da Blendquellen nahe dem Punkt, auf den der Blick gerichtet ist, eine höhere Störwirkung auf das Auge haben als periphere Blendquellen. Folglich können helle Punkte mit zunehmender Entfernung vom Punkt, auf den der Blick gerichtet ist, weniger stark gewichtet werden. Zur Implementierung dieses Berechnungsverfahrens ist eine Ermittlung der Blickrichtung notwendig, was beispielsweise durch die Pupillenerfassungseinrichtung erfolgen kann. Eine einfachere Möglichkeit, diese Ortsabhängigkeit in die Berechnung einfließen zu lassen, ohne eine Blickrichtungsverfolgung zu benötigen, ist, einen starren Blick geradeaus anzunehmen. Da die Blickrichtung beim Menschen meist annähernd geradeaus gerichtet ist und sonst der Kopf bewegt wird, deckt diese vereinfachende Annahme viele Fälle ab.

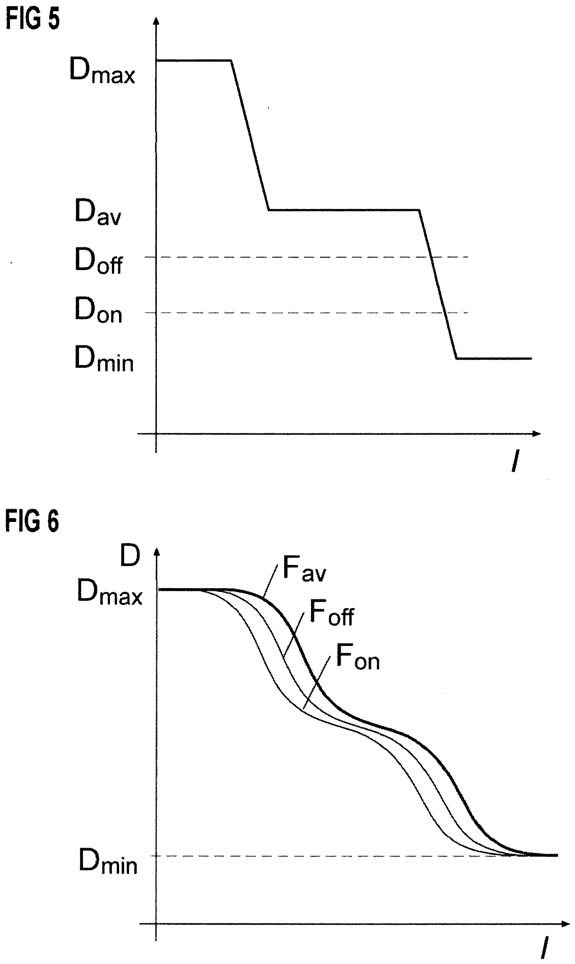

Fig. 5 zeigt ein Ausführungsbeispiel für die zuvor beschriebene Anpassungsfunktion der Blendschutzbrille, wobei ein konstanter durchschnittlicher, d.h. über eine oder mehrere Sekunden gemittelter, Pupillendurchmesser vorausgesetzt wird. Dabei ist auf der horizontalen Achse die Helligkeit I aufgetragen und auf der vertikalen Achse der Pupillendurchmesser D. Weiter bezeichnen Dₘₐₓ den maximalen Pupillendurchmesser des Trägers der Blendschutzbrille und Dₐᵥ den durchschnittlichen Pupillendurchmesser des Trägers nach vollständiger Helligkeitsadaption in einem mittleren Helligkeitsbereich zwischen den extremen Helligkeiten. Dₘᵢₙ bezeichnet den minimalen Pupillendurchmesser des Trägers der Blendschutzbrille.

D_{off} bezeichnet die Abschaltschwelle der Abdunklungsfunktion. Wenn der Pupillendurchmesser D über diesen Wert steigt, wird die Abdunklung abgeschwächt oder ganz abgeschaltet. D_{off} liegt unter Dₐᵥ, um einen permanenten Adaptionsdruck auf die Netzhaut auszuüben. Die Netzhaut wird dann weiter versuchen, sich an die größere Helligkeit anzupassen. Wenn ihr das gelingt, wird die Abdunklungsfunktionalität der Brille weniger wichtig und kann reduziert bzw. abgeschaltet werden. Damit erreicht die Brille wieder ihren vollen Spielraum zur Abdunklung, falls es noch heller wird.

Dₒₙ ist die Anschaltschwelle der Abdunklungsfunktion. Wenn der Pupillendurchmesser D unter diesen Wert fällt, wird die Abdunklung eingeschaltet oder verstärkt, falls sie bereits eingeschaltet ist und noch nicht an der Obergrenze ihrer Leistungsfähigkeit liegt.

Die Werte Dₘₐₓ, Dₐᵥ und Dₘᵢₙ können vor der ersten Benutzung der Brille manuell parametriert werden. Die Brille kann aber auch derart eingerichtet sein, dass die entsprechenden Parameter automatisch bestimmt werden, indem über einen längeren Zeitraum von der Pupillenerfassungseinrichtung ermittelten Werte des Pupillendurchmessers des Trägers aufgenommen und ausgewertet werden. Dₘₐₓ und Dₘᵢₙ werden entsprechend den Werten des Pupillendurchmessers D angenommen, die maximal bzw. minimal in einem bestimmten Zeitraum, beispielsweise innerhalb der letzten Woche, gemessen wurden. Die automatische Messung dieser Extremwerte kann auch forciert werden, indem der Träger plötzlich in ein helles Licht blickt und dann die Brille abdeckt, ohne die Lider zu schließen.

Dₐᵥ kann gemessen werden, wenn die Helligkeit über einen Zeitraum von beispielsweise einer halben Stunde annähernd konstant und nicht im extremen Bereich war. Diese dynamische Parametrierung kann eine automatische Anpassung an Änderungen beim Träger bewirken. Dabei sollte möglichst keine sofortige Änderung von Dₐᵥ (für eine bestimmte absolute Helligkeit) erfolgen, weil der Wert beispielsweise im Tagesverlauf schwanken kann. Bei Müdigkeit oder auch bei Erschöpfung, in Zusammenhang mit vielen Krankheiten, werden die Augen lichtempfindlicher und die Pupillen verkleinern sich. Dann ist es für den Träger angenehm, wenn die nun permanent kleineren Pupillen als Hinweis auf den Wunsch nach früherer Abdunklung dienen und die Gläser bei geringerer Helligkeit abgedunkelt werden. Es ist daher vorteilhaft, die gespeicherten und verwendeten Dₐᵥ - Werte mit einer Verzögerung von mehreren Tagen an erkannte Änderungen anzupassen.

Die Werte Dₒₙ und D_{off} können in besonders einfacher Weise dadurch eingestellt werden, dass der Bereich zwischen Dₐᵥ und Dₘᵢₙ durch Dₒₙ und D_{off} in drei gleiche Teile aufgeteilt wird, wie beispielhaft in Fig. 5 gezeigt. Diese Verhältnisse können auch manuell parametrierbar gemacht werden.

Fig. 6 zeigt ein verfeinertes Ausführungsbeispiel für die Ermittlung der Einschalt- und der Abschaltschwelle der Abdunklung anhand des Pupillendurchmessers, wobei die Achsen wie in Fig. 5 gewählt sind. Dabei wird jedoch nicht von einem im Wesentlichen konstanten Wert für Dₐᵥ ausgegangen, sondern von einer komplizierteren Abhängigkeit des Pupillendurchmessers von der Helligkeit nach vollständiger Adaption. Dₘₐₓ und Dₘᵢₙ bezeichnen auch in Fig. 6 den maximalen und den Minimalen Pupillendurchmesser des Trägers der Blendschutzbrille.

Fₐᵥ ist eine Funktion der Helligkeit I, die den durchschnittlichen Pupillendurchmesser Dₐᵥ des Trägers der Blendschutzbrille nach vollständiger Adaption an die jeweilige Helligkeit beschreibt. Ggf. auftretende rasche Schwankungen des Pupillendurchmessers (Hippus) sind dabei durch die Mittelung eliminiert.

F_{off} ist eine Funktion, die die Abschaltschwelle der Abdunklungsfunktion in Abhängigkeit von der Helligkeit I beschreibt. In einer einfachen Ausführung kann angenommen werden, dass F_{off} um einen konstanten Wert oder einen konstanten Faktor gegenüber Fₐᵥ zu kleineren Helligkeitswerten versetzt ist. Wenn der Pupillendurchmesser D über diesen Wert steigt, wird die Abdunklung abgeschwächt oder abgeschaltet.

Fₒₙ bezeichnet dementsprechend eine Funktion, die die Anschaltschwelle der Abdunklungsfunktion in Abhängigkeit von der Helligkeit I beschreibt. In einer einfachen Ausführung kann Fₒₙ um einen konstanten Wert oder einen konstanten Faktor gegenüber F_{off} zu kleineren Helligkeitswerten versetzt angenommen werden. Wenn der Pupillendurchmesser D unter diesen Wert fällt, wird die Abdunklung eingeschaltet oder verstärkt, falls sie bereits eingeschaltet ist und noch nicht an der Obergrenze ihrer Leistungsfähigkeit liegt.

Auch eine Anwendung beider oben genannter Ausführungsbeispiele in derselben Brille ist möglich: Das Verfahren, das mit Fig. 5 beschrieben ist, kann etwa zur anfänglichen Anpassung an einen neuen Träger verwendet werden. Wenn im täglichen Einsatz viele Messpunkte des Trägers von Fₐᵥ aufgenommen worden sind, kann die Blendschutzbrille zum Verfahren nach Fig. 6 übergehen.

Die Auswertung des Pupillendurchmessers zur Ermittlung des Abdunklungsbedarfs ist bei jungen Menschen leichter als bei älteren. Grund ist die sich mit zunehmendem Alter verringernde maximale Öffnung der Pupille. Der Durchmesser der Pupille muss bei älteren Trägern also viel genauer gemessen werden, um den Abdunklungsbedarf zuverlässig ermitteln zu können. Je älter der Träger ist, um so wichtiger werden andere Indizien zur Ermittlung der nötigen Intensität der Abdunklung. Die nachfolgend beschriebenen Auswertungen der Mimik des Trägers können hier einspringen.

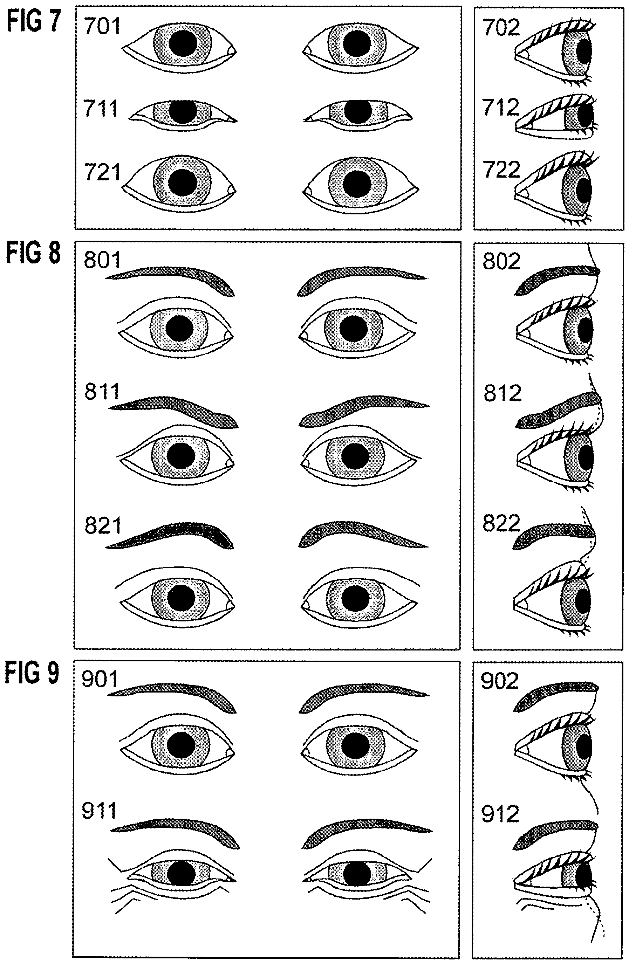

Die Augenkameras können dazu verwendet werden, einen Teil der Mimik des Trägers zu erkennen. Beispiele für eine solche erfassbare Mimik sind in den Figuren 7 bis 9 dargestellt. Erkannte Mimikreaktionen können zur Anpassung der Anschaltschwelle bzw. der Intensität der Abdunklung verwendet werden. Neben der Steuerung einer Blendschutzfunktionalität können die hier beschriebenen Mimikerkennungen auch zur Steuerung weiterer Funktionalitäten, vor allem im Zusammenhang mit einer Datenbrille, verwendet werden. In den folgenden Ausführungsbeispielen ist mehrfach von einem entspannten Zustand die Rede. Dieser Zustand kann von der Steuerelektronik mit statistischen Methoden aus immer wieder längere Zeit gehaltenen Durchschnittswerten für jede Person ermittelt werden. Eine aktive Parametrierung von einem Menschen ist dafür also nicht erforderlich, wohl aber möglich.

Fig. 7 zeigt beispielhaft Bewegungen der Augenlider beider Augen, die erfasst werden können, nämlich die Augenlider im entspannten Zustand (701), die Augenlider weiter geschlossen (711) als im entspannten Zustand und die Augenlider weiter geöffnet (721) als im entspannten Zustand. Ein weiter geschlossener Zustand kann als Indiz gewertet werden, dass es dem Träger zu hell ist. Daraufhin veranlasst die Steuerelektronik eine stärkere Abdunklung. Bei weiter geöffneten Augenlidern wird eine eventuelle Abdunklung etwas zurückgenommen.

Zur Erfassung der Bewegungen der Augenlider können die Pupillenvermessungskameras genutzt werden, die die Pupillen beobachten und vermessen. Dabei wird die Linie des inneren Irisrands vermessen. Die Augenlider ragen bei diesen Vermessungen immer wieder über die Iris und müssen deshalb sowieso berücksichtigt werden. Zur Beachtung der Bewegungen der Augenlider werden die Innenkanten der Lider im erfassten Bild nun systematisch berechnet und ausgewertet. Alternativ kann auch der Körperumriss ausgewertet werden. Wenn die Augenkameras extrem seitlich des Auges auf der Brille angebracht sind, sind die Lider und der Augapfel gut aus dem Körperumriss erkennbar (702, 712, 722). Dabei kann für die Weiterverarbeitung diese Information zu einer Helligkeitssteuerung der Brille die Abweichung vom Ruhezustand und/oder der Grad der Überdeckung der Pupille genutzt werden. Der zweite Fall kann als intensiverer Bedarf nach Abdunklung interpretiert werden und eine schnellere und intensivere Abdunklung auslösen.

In Fig. 8 sind beispielhaft Bewegungen der Augenbrauen gezeigt, nämlich die Augenbrauen im entspannten Zustand (801), die Augenbrauen gesenkt (811) und die Augenbrauen gehoben (821). Die Abbildungen zeigen jeweils das rechte und das linke Auge von vorn (801, 811, 821) .Danach ist jeweils das linke Auge aus Sicht einer Kamera, die sich auf dem Nasenrückenbefindet, abgebildet (802, 812, 822). Auf diesen letzten Abbildungen sind die Verschiebung der Auswölbung der Augenbrauen als Verschiebung des Körperumrisses nach unten (812) bzw. nach oben (822) dargestellt. Die gestrichelte Linie in 812 und 822 entspricht dem Ruhezustand. Erkannte gesenkte Augenbrauen können als verstärktes Bedürfnis des Trägers nach Abdunklung gewertet werden, so dass entsprechend mehr oder schneller abgedunkelt wird. Umgekehrt kann das Anheben der Augenbrauen als Wunsch nach mehr Licht verstanden werden und die Abdunklung daraufhin zurückgenommen werden.

Zur Erkennung der Bewegungen der Augenbrauen kann es vorgesehen sein, dass die Kameras an dem Brillengestell einen größeren Bereich als nur die Iris erfassen. Dies können die Augenkameras der Pupillenerfassungseinrichtung sein oder zusätzliche Kameras. Drei Parameter können zur Ermittlung von Augenbrauenbewegungen verwendet werden:
(1) Die Ausgeprägtheit bzw. die Dunkelheit und Breite der Falte über den Augenlidern, vor allem in den äußeren, d.h. von der Nase weggewandten, zwei Dritteln dieser Falte.
(2) Die Positionsänderung von Strukturen über dem Auge, insbesondere der Haare der Augenbrauen oder Hautstrukturen. Hier ist es vor allem in Richtung zur Nase gewandte Teil der Augenbrauen, der zur Ermittlung der Augenbrauenbewegungen verwendet werden kann.
(3) Von der Seite sichtbare Verschiebungen der Hautauswölbungen der Augenbrauen. Wenn die Augenkameras extrem seitlich des Auges auf der Brille angebracht sind, ist der Körperumriss gut erkennbar. Dieser Körperumriss verändert sich bei Bewegungen der Augenbrauen.

In Fig. 9 ist die Anhebung der Wangen gezeigt, nämlich die Wangen im entspannten Zustand (901) und die Wangen Richtung Auge gehoben (911). Die Abbildungen zeigen jeweils das rechte und das linke Auge von vorn (901, 911). Danach ist jeweils das linke Auge aus Sicht einer Kamera, die sich auf dem Nasenrücken befindet, abgebildet (902, 912). Auf diesen letzten Abbildungen sind die Verschiebung der Auswölbung der Wange als Verschiebung des Körperumrisses nach oben (912) gegenüber dem Ruhezustand (902) dargestellt. Die gestrichelte Linie in 912 entspricht dem Ruhezustand. Auch gehobene Wangen können als Bedürfnis des Trägers nach Abdunklung verwendet werden und zur weiteren Abdunklung führen.

Das Erkennungskriterium zur Erfassung der Anhebung der Wangen sind ausgeprägtere nach außen gehende Falten (dunkle Linien) unter dem Auge als im entspannten Zustand. Sie können besser von einer außen liegenden Kamera als von einer Kamera an der Nase erkannt werden.

Die bisher genannten mimischen Bewegungen im Augenbereich können als Standardsteuerung der Blendschutzbrille verwendet werden. Einzelne dieser mimischen Bewegungen können auch als erlernbare Kommandos implementiert werden.

Ein Zusammenkneifen der Augen kann allerdings auch Folge von Wind sein, beispielsweise Fahrtwind, der die Augen reizt. Daten von einem Windsensor 105 (s. Fig. 1) können derartige Fehlinterpretationen reduzieren helfen. Im gegebenen Fall würde die Auswertung der Mimik abgeschaltet oder in ihrer Auswirkung auf die Abdunklung abgeschwächt werden.

Des Weiteren können Fehlinterpretationen entstehen, wenn die Mimik als menschliches Kommunikationsmittel verwendet wird und nicht als Ausgleich einer wahrgenommenen zu großen oder zu geringen Helligkeit. Eine Erkennung solcher Kommunikationssituationen könnte verwendet werden, um eine Anpassung der Abdunklung aufgrund der Mimik vorübergehend abzuschalten oder abzuschwächen. Entsprechende Situationen könnten optisch anhand erkannter Personen bzw. Gesichtern im Erfassungsbereich der Szenekamera oder akustisch, anhand von erkannten Gesprächen, identifiziert werden. Zur Implementierung des ersten Falls kann das Bild der Szenekamera in ähnlicher Weise, wie es bei Fotokameras bekannt ist, zur automatischen Erkennung der Anwesenheit eines Gesichts bzw. einer Person im Nahbereich ausgewertet werden. Für den zweiten Fall können Signale eines Mikrofons auf der Brille ausgewertet werden, wie dies etwa bei Telefonen und Headsets bekannt ist. Bei einer Kombination mit einer Datenbrille könnten solche Teilfunktionalitäten ebenfalls genutzt werden.

Durch Speicherung der Anpassungsaktionen der Abdunklung anhand der Mimik des Trägers über einen längeren Zeitraum können individuelle Adaptionskurven des Trägers angepasst werden. Damit parametriert sich die Brille teilweise selbst.

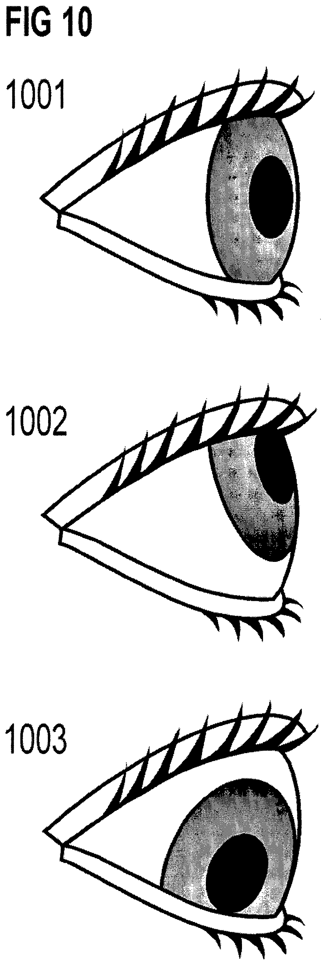

In Fig. 10 ist schematisch das Bild, das eine Augenkamera aus weit außen liegender Position vom Auge aufnimmt, gezeigt, und zwar bei geradeaus gerichtetem Blick (1001), bei nach links oben gerichtetem Blick (1002) und bei nach rechts unten gerichtetem Blick (1003). Die Positionsbestimmung der Pupille kann in an sich bekannter Weise durch Eye-Tracking erfolgen.

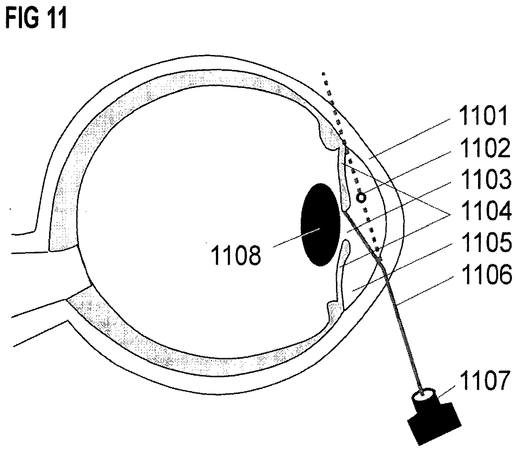

Bei den Berechnungen ist darauf zu achten, dass es sich beim Auge, speziell im Bereich der Pupille, nicht um eine Kugel handelt, wie in Fig. 11 schematisch dargestellt ist. Die Hornhaut 1101 ist über die Iris 1104 und die Pupille 1103 nach außen gewölbt, während die Iris 1104 annähernd flach ist. Dabei kann diese Wölbung über der vorderen Augenkammer 1105 zu Verzerrungen beim Blick der Augenkameras von der Seite auf die beiden Begrenzungslinien der Iris 1104 durch die Lichtbrechung führen. Von der Augenkamera 1107 aus erscheint die innere Begrenzungslinie der Iris, also der Pupillenrand, in Verlängerung des Strahls 1106, nämlich im Punkt 1102. Die Positionsbestimmung und die Durchmesserermittlung der Pupille 1103 werden besser, wenn solche Effekte von der Steuerelektronik herausgerechnet werden. Mit dem Bezugszeichen 1108 ist die Linse bezeichnet. Ein Anpassen der Brille an den Träger mit seinen individuellen Augen vor der ersten Benutzung kann sinnvoll sein.

Zur verbesserten Erfassung der Augenbewegungen können für jedes Auge weitere Augenkameras beispielsweise an der Innen- und an der Außenseite der Brille hilfreich sein. Wenn das Auge beispielsweise nach links gerichtet ist, kann die Pupillengröße- und Position mit Hilfe der links angebrachten Augenkameras berechnet werden und umgekehrt bei einer Blickrichtung nach rechts mit der rechts angeordneten Augenkamera. Eine Erfassung mit mehreren Kameras gleichzeitig ist noch genauer und weniger störanfällig.

Beide menschlichen Augen sind normalerweise synchron auf einen Punkt gerichtet. Diese Annahme kann verwendet werden, wenn die Pupillenposition eines Auges zuverlässig erkannt werden kann, die des anderen Auges jedoch nicht. Dann wird die wahrscheinliche Position der Pupille auf dem zweiten Auge aus den Daten des ersten errechnet. Solche Fälle können beispielsweise auftreten, wenn nur innen im Bereich der Nase Augenkameras angeordnet sind, die Augen extrem nach rechts oder links gerichtet sind oder die Bilderkennung durch Störungen erschwert ist. Beim Rückschluss von einer Augenposition auf die andere ist jedoch auch eine Annahme der Entfernung des betrachteten Objekts nötig. Grobe Abweichungen der Entfernung können zu falschen Ergebnissen zur Ansteuerung einer Abdunklung des Glases des anderen Auges führen. Um die Entfernung eines betrachteten Punktes zu ermitteln, sind die Einbindung von Verfahren zur räumlichen Szenenerkennung und eine echte Blickverfolgung vorteilhaft. In vereinfachter Weise kann andererseits eine unendliche Entfernung des Objekts angenommen werden.

Ferner kann in manchen Fällen eine Berücksichtigung der Lichtbrechung in den Gläsern der Blendschutzbrille bei der Berechnung von Position, Form und Größe der abzudunkelnden Flächen der Gläser notwendig sein, insbesondere dann, wenn eine Blendquelle weit von der Sehachse entfernt ist.

Bei der Nutzung des Pupillendurchmessers ist dessen natürliche Oszillation (Hippus) zu berücksichtigen, bei der sich 2 bis 3 mal pro Sekunde der Pupillendurchmesser etwas ändert. Die Brille muss dabei nicht in dieser hohen Frequenz die abgedunkelten Bereiche auf der Brille den kleinen Durchmesseränderungen der Pupille anpassen, da dieses "Pumpen" nicht bewusst wahrgenommen wird. Hierfür kann ein Tiefpassfilter bei der Auswertung des Pupillendurchmessers vorgesehen sein. Ein Tiefpassfilter vor der Auswertung des Pupillendurchmessers hilft auch, menschliche Reflexe auszugleichen, wie den Lidschluss- und den Dunkelheitsreflex.

In vielen Fällen wird die beobachtete Szenerie in Bewegung sein. Vom Beobachter aus gesehen werden blendende Objekte und Flächen sich auf bestimmten Bahnen bewegen. Da die Szenenerfassung, die Berechnung der Flächen auf der Brille, die abgedunkelt werden sollen, sowie die Ansteuerung der Abdunklung der Gläser Zeit benötigen, kann dies bei schnellen Objektbewegungen zu Abdunklungen an den falschen Stellen führen. Diese Verzögerung kann ausgeglichen werden, indem die Bewegungen der Objekte vorausberechnet werden. Die Steuerelektronik berechnet den Ort in der Szene, an denen sich die Objekte befinden werden, um den Zeitraum der Verzögerung in der Zukunft versetzt. Dann wird die Brille an den entsprechenden, in der Zukunft nötigen Stellen abgedunkelt.

Die Abdunklungen müssen auf die beobachtete Szene bezogen sein. Auch bei Bewegungen des Kopfes und damit der Brille sollten die abgedunkelten Flächen so auf den Gläsern wandern, dass deren Schatten immer in die Pupille fällt. Dies kann ähnlich implementiert werden wie zuvor für Objektbewegungen beschrieben. Allerdings ist der Bezugsrahmen ein anderer. Beim Ausgleich der Kopfbewegungen wandert die gesamte Szene. Es müssen die Punkte um den Verzögerungszeitraum in der Zukunft für alle abzudunkelnden Objekte der Szene gleichmäßig berechnet werden. In einer ersten Annäherung kann das ganze erfasste Bild vor der Weiterverarbeitung um ein bestimmtes Maß verschoben werden. Präzisere Ergebnisse liefern Berechnungen in Abhängigkeit von der Blick- und der Brillenachse. Die einfache Verschiebung reicht aus, wenn die Reaktionsträgheit der Brille nicht zu groß ist.

Kopfbewegungen können optisch erkannt werden, wenn in aufeinanderfolgende Bilder der Szenekamera gleichmäßig verschobene Bilder erkannt werden, und/oder durch Auswertung eines optionalen Beschleunigungssensors 106 (s. Fig. 1). Werden mehrere Beschleunigungssensoren auf der Brille verteilt eingesetzt, können Bewegungen in mehreren Achsen besser erkannt werden. Die optische Auswertung und auch die Beschleunigungssensoren können die Steuerelektronik jedoch täuschen. Deshalb ist eine Auswertung beider genannten Indizien hilfreich, wobei ggf. eine Gewichtung gemäß einer Sicherheit der Erkennung einer Beschleunigung den Ausgleichsberechnungen zugrunde gelegt werden kann.

Da das Ziel der Abdunklungen nicht das ganze Auge, sondern die Pupille ist, ist es vorteilhaft, auch Bewegungen bzw. Drehungen des Auges auszugleichen. Auch hier ist es hilfreich, wenn die Reaktionsträgheit der Brille in die Berechnungen einbezogen wird. Der Ausgleich der Augenbewegungen kann ähnlich implementiert werden wie zuvor für Kopfbewegungen beschrieben, nur dass die Verschiebung nicht auf die Lichtquellen, die Szenerie, bezogen berechnet wird, sondern auf das Ziel, die Pupille.

Die oben erwähnte Reaktionsträgheit der Blendschutzbrille kann bei rasch veränderlichen Blendquellen dazu führen, dass eine Abdunklung erst erfolgt, wenn die Lichtquelle schon wieder verschwunden ist, und eine Aufhellung, wenn bereits ein weiterer Blitz eintrifft. Bewegen sich beispielsweise die Scheinwerfer eines blendenden, entgegenkommenden Autos aus Sicht des Trägers der Brille hinter den Pfosten einer Mittelleitplanke vorbei, kann eine Folge von Lichtblitzen entstehen, der die Brille nicht in Echtzeit folgen kann. Als Ausgleich für eine in diesem Fall zu geringe Reaktionsgeschwindigkeit ist ein verzögertes Zurücknehmen der Abdunklung möglich. Reduziert die Brille die Abdunklung über einen Zeitraum von beispielsweise einer Sekunde, so kann das Aufblitzen der Scheinwerfer des entgegenkommenden Autos durchgängig aktiv abgedunkelt werden.

Um zu verhindern, dass Objekte so vollständig abgedunkelt werden, dass der Träger der Brille sie nicht mehr wahrnehmen kann, kann beispielsweise die Abdunklungsfähigkeit der Brille bzw. der maximal erreichbare Abdunklungsgrad dadurch begrenzt werden, dass das Raster der LCD-Elemente so ausgelegt wird, dass zwischen den Pixeln nicht abdunkelbare Lücken verbleiben. Ist das Verhältnis der Flächen der Pixel zu den Lücken beispielsweise neun zu eins, kann der maximale Abdunklungsgrad nicht größer als 90% sein. Abgedunkelte Bereiche werden also mindestens noch zu 10% sichtbar sein. Eine Auslegung der LCD-Schicht derart, dass im abgedunkelten Zustand noch 10% Transmission besteht, hat denselben Effekt. Diese Maßnahme entspricht gesetzlichen Vorgaben in manchen Ländern, die fordern, dass Sonnenbrillen, die im Straßenverkehr getragen werden nicht stärker als ein bestimmtes Maß sein dürfen.

Es kann alternativ oder zusätzlich hierzu auch vorgesehen sein, dass die Abdunklung in peripheren Bereichen der Brille geringer ist. Dabei wird ausgenutzt, dass die menschliche Wahrnehmung außerhalb des mit dem Blick fixierten Bereichs ist geringer ist und auch die subjektive Blendwirkung in peripheren Bereichen des Gesichtsfelds geringer ist. Hierfür kann die Steuerungseinrichtung so eingerichtet sein, dass bezogen auf die Blickrichtung periphere Blendungen weniger stark abgedunkelt werden, insbesondere ein geringerer Abdunklungsgrad oder eine geringere Größer der abgedunkelten Fläche gewählt wird, und/oder es kann das Pixelraster in den Randbereichen der Gläser, insbesondere in den seitlichen Bereichen, weniger dicht ausgelegt sein als in den zentralen und ggf. oberen Bereichen.

Weiter alternativ oder zusätzlich kann zur Vermeidung, dass Objekte durch Abdunklung nicht mehr wahrnehmbar werden, die Steuerelektronik so ausgelegt sein, dass kleine Objekte erst ab einem Mindestkontrast zur restlichen Szene und nicht zu stark abgedunkelt werden.

Um die Blendschutzbrille auch als gewöhnliche Sonnenbrille verwenden zu können, kann es vorgesehen sein, dass über einen Schalter eine gleichmäßige flächige Tönung einer vorbestimmbaren Stärke fest eingeschaltet wird. Der Schalter kann mechanisch auf der Brille, über die drahtlose Steuerung von dem externen Gerät oder akustisch über Sprachbefehle bedient werden. Diese Zuschaltung kann in mehreren Stufen möglich sein. Wenn diese fest eingestellte Tönung nicht bereits den maximal möglichen Abdunklungsgrad der Brille darstellt, kann die Blendschutzbrille weiterhin mit noch stärkerer Abdunklung für helle Objekte die feste Tönung überlagern. Sollte die Helligkeit der Szene so weit absinken, dass die manuell eingestellte Tönung zu stark wird, kann die Brille diese Tönung automatisch teilweise oder ganz zurücknehmen; auch ein teilweises Zurücknehmen einer flächiger Abdunklung ist möglich, wenn beispielsweise ein großer Bereich im Vordergrund, wie das Armaturenbrett eines Autos, sehr dunkel ist. Später, wenn die Umgebungshelligkeit wieder so weit angestiegen ist, dass eine flächige Abdunklung noch ausreichende Sicht ermöglicht, kann die Abdunklung wieder automatisch zugeschaltet werden.

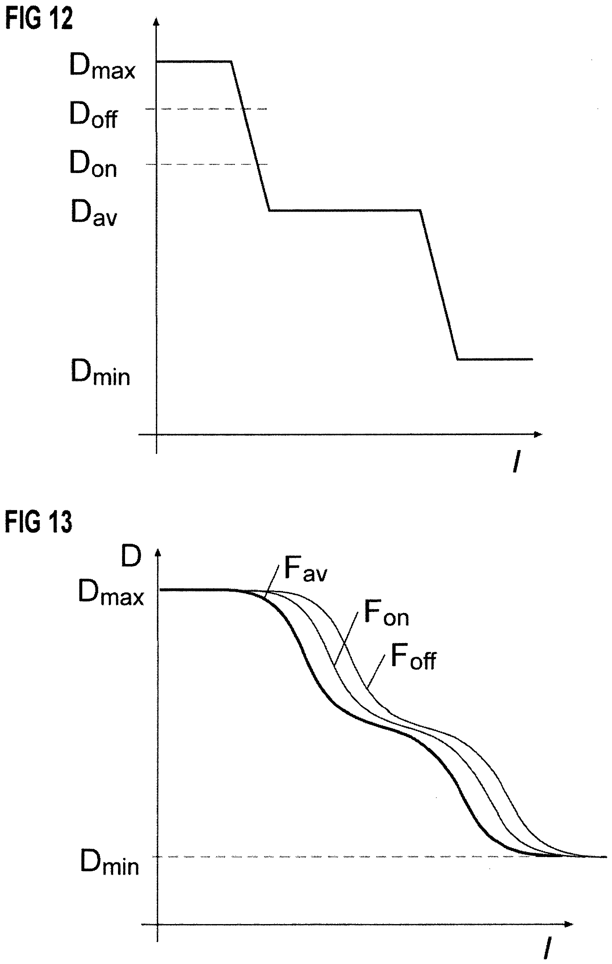

Fig. 12 zeigt eine Möglichkeit, die Einschalt- bzw. Verstärkungsschwelle der Sonnenbrillenfunktion, d.h. der gleichmäßigen Abdunklung, anhand des Pupillendurchmessers D zu ermitteln; dies stellt eine Abwandlung des Verfahrens mit dem als konstant angenommenen durchschnittlichen Pupillendurchmesser Dₐᵥ dar, das mit Fig. 5 erläutert wurde. Die Schwellwerte D_{off} und Dₒₙ werden zwischen Dₘₐₓ und Dₐᵥ gelegt, statt zwischen Dₐᵥ und Dₘᵢₙ.

Fig. 13 zeigt eine weitere Möglichkeit, die Einschalt- bzw. Verstärkungsschwelle der Sonnenbrillenfunktion anhand des Pupillendurchmessers zu ermitteln. Es wird eine Abwandlung des Verfahrens mit dem komplexeren Verlauf der durchschnittlichen Pupillenweite verwendet, das mit Fig. 6 erläutert wurde. Die Funktionen F_{off} und Fₒₙ sind als Verschiebungen von Fₐᵥ nach rechts statt nach links eingestellt.

Vereinfachte Verfahren, insbesondere vereinfachte Berechnungsverfahren, können helfen, die Baugröße, die Entwicklungskosten und die Herstellungskosten zu verringern. Außerdem könnte der Stromverbrauch verringert bzw. die Batterielaufzeit verlängert werden. Vereinfachende Annahmen bzw. Verfahren könnten sein:
- Die Brille als Ebene annehmen (obwohl Brillen normalerweise etwas konvex sind);
- die Pupille als Kreis auf einer Ebene parallel zur Brillenebene annehmen (obwohl sie, je nach Augenausrichtung unterschiedlich geneigt ist);
- die betrachtete Szenerie in unendlicher Entfernung annehmen (bei einer Entfernung von ca. 2cm zwischen Auge und Brille, ca. 5cm zwischen Szenekamera und Augen, sowie Entfernungen von mindestens 10m zu den blendenden Objekten wird der Fehler bei der Berechnung vernachlässigbar klein);
- die Helligkeitsermittlung nicht über die Szenekamera 103 (s. Fig. 1), sondern über den Helligkeitssensor 109, wobei die Szenekamera 103 dann ausgeschaltet wird, wenn die ermittelte Helligkeit in einem vorgebbaren Bereich liegt (dies hat den Vorteil, dass ein einfacher Helligkeitssensor einen geringeren Stromverbrauch hat als eine Kamera sowie dass keine energieintensive Bildverarbeitung der Kamerabilder nötig ist; sobald die vom Helligkeitssensor 109 gemessene Helligkeit den vorgebbaren Bereich über- oder unterschreitet, wird die Szenekamera 103 wieder eingeschaltet).

Vereinfachte Berechnungsverfahren können - situationsabhängig - auch mit aufwendigeren abgewechselt werden, um den Energiebedarf der Brille zu senken. Anhand des von der Szenekamera erfassten Bildes kann eine Situation automatisch als typisch für den Nahbereich oder typisch für den Fernbereich klassifiziert werden. In Fernbereichssituationen, evtl. mit ruhigem Blick geradeaus, könnte die vereinfachte Berechnung angewandt werden, da aufwendigere Verfahren keine wesentliche Qualitätsverbesserung der Abdunklung bringen.

Ein zweiter Weg Energie zu sparen ist, Sensoren zur Pupillenbeobachtung oder auch Mikrofon und Beschleunigungssensor abzuschalten bzw. in niedrigeren Frequenzen auszuwerten, wenn sie nicht benötigt werden. Dies kann beispielsweise sinnvoll sein, wenn keine Abdunklung nötig ist. Dann müssen nur die Szenekamera und die Auswertung des aufgenommenen Bilds der Szenekamera aktiv bleiben, um zu ermitteln, ob die Abdunklungsfunktion wieder benötigt wird. In diesen Phasen kann auch die Auswertung der Bilder der Szenekamera in größeren Abständen durchgeführt werden. Es könnte ausreichen, in diesem Standby-Modus beispielsweise jede Sekunde ein Bild auszuwerten. Damit reduziert sich der Energieverbrauch drastisch. Auch bei den anderen Sensoren könnte eine Reduktion der Auswertefrequenz situationsabhängig sinnvoll sein, um Energie zu sparen. Sollte die Auswertung der Bilder der Szenekamera Blendungen vermuten lassen, kann die Pupillengrößenmessung und/oder die Mimikerkennung zugeschaltet werden, um die Entscheidung, ob eine Abdunklung erfolgen soll, zu unterstützen. Auch die Szenekamera kann bei konstanten Lichtverhältnissen abgeschaltet werden, wenn entweder keine oder eine ganzflächige Abdunklung aktiviert ist. Es wird dann nur noch über den Helligkeitssensor 109 (s. Fig. 1) überwacht, ob größere Helligkeitsschwankungen auftreten.

In einfachen Blendungssituationen ohne stark heraus leuchtende Objekte - also bei relativ gleichmäßiger, aber hoher Helligkeit - kann auch eine großflächige Tönung der Gläser eingeschaltet werden, die keine Pupillenvermessung und schnelle Verfolgung von Objekten mit der Szenekamera erfordert. Das restliche System kann dann in den Standby-Modus geschaltet werden, wie oben beschrieben. Eine solche großflächige Tönung kann beispielsweise eine ganzflächige Abdunklung (wie bei einer normalen Sonnenbrille), eine in der oberen Hälfte höhere Abdunklung (wie bei bekannten halbgetönten Sonnenbrillen) oder eine großflächige einseitige Abdunklung sein.

Oben sind einige Funktionalitäten beschrieben, bei denen die Blendschutzbrille an eine bestimmte Person angepasst wird bzw. bei denen die Blendschutzbrille Eigenheiten des Trägers lernt. Wenn eine andere Person dieselbe Blendschutzbrille verwenden möchte, würde die Brille bei der zweiten Person schlecht funktionieren, weil diese Werte nicht passen. Die selbst lernenden Funktionen würden, solange die zweite Person die Brille trägt, Daten der zweiten Person lernen, die die Daten der ersten Person überschreiben und die Anpassung daher verschlechtern. Es kann deshalb vorgesehen sein, dass die Lernfunktionen deaktiviert sind, wenn die Brille von einer anderen Person als einem vorgebbaren primären Träger benutzt wird, oder dass individuelle Daten für mehrere Personen getrennt gespeichert werden. Beide Varianten können entweder manuell oder automatisch umgesetzt werden. Zur manuellen Umsetzung kann ein Schalter zum Abschalten des Lernens bzw. zum Umschalten der Personen vorgesehen sein. Eine automatische Umschaltung kann anhand biometrischer Daten erfolgen, wofür manche der Daten genutzt werden, die die Brille sowieso erfasst. Diese Daten können durch Klassifikation Personen unterscheiden helfen.

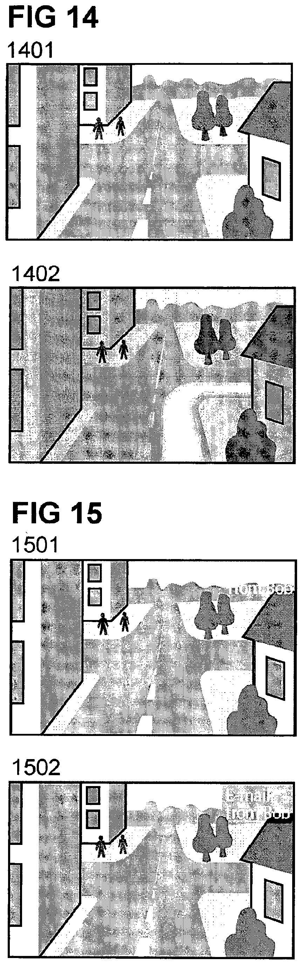

Eine erfindungsgemäße Blendschutzbrille kann gemäß einem weiteren Ausführungsbeispiel auch Augmented-Reality-Funktionen aufweisen. Im oberen Bild der Figur 14 ist beispielhaft eine Szene dargestellt, wie diese ohne Aktivierung der Augmented-Reality-Funktion erscheint (1401). Im unteren Bild der Figur 14 ist ein Weg, der beispielsweise von einem Navigationssystem vorgeschlagen wird, hervorgehoben, indem im Bereich des vorgeschlagenen Wegs, vom Auge aus gesehen, keine Abdunklung erfolgt, während die Umgebung leicht abgedunkelt wird (1402). Ein Teil des wahrgenommenen Bildes wirkt dabei wie durch eine Sonnenbrille betrachtet, während hervorzuhebende Bildteile heller wirken, in diesem Fall ein Teil der Straße. In einem weiteren Anwendungsfall kann beispielsweise ein Zielgebäude heller dargestellt werden als die Umgebung. Die Information, welche Bildteile heller und welche dunkler erscheinen sollen, wird dabei von einem externen Augmented-Reality-System an die Blendschutzbrille übertragen. Die Steuerungseinrichtung der Blendschutzbrille berechnet aus diesen Informationen die abzudunkelnden Bereiche und steuert die Gläser entsprechend an.

Die Blendschutzbrille kann auch Datenbrillenfunktionen aufweisen, wobei einige Teilfunktionalitäten auch hierfür genutzt werden können. Mit Datenbrillen sind Brillen gemeint, bei denen dem Träger Texte und/oder Bilder in sein Gesichtsfeld eingeblendet werden, Videobrillen, Untertitelbrillen, Telefonbrillen, Internet-Surf-Brillen sowie Brillen, die zum freihändigen Fotografieren und Filmen bestimmt sind; auch Augmented-Reality-Brillen können Datenbrillenfunktionen aufweisen.

Wie beispielhaft in Fig. 15 gezeigt ist, kann eine erfindungsgemäße Blendschutzbrille gemäß einem Ausführungsbeispiel zur Unterstützung von Einblendungen in durchsichtige Augmented-Reality-Brillen (see through augmented reality glasses) verwendet werden. Bei durchsichtigen Augmented-Reality-Brillen können Bilder, Text und/oder Zeichen in das Sichtfeld eingespiegelt werden. Dabei wird nach Art eines Projektors zusätzliches Licht eingeblendet. Die Erkennbarkeit der Einblendung wird durch die Helligkeit des Hintergrunds begrenzt. Ein solcher Fall ist beispielhaft im oberen Bild der Figur 15 dargestellt, worin aufgrund des hellen Hintergrunds nur ein Teil einer eingeblendeten Schrift lesbar ist (1501). Wie in dem unteren Bild dargestellt ist, können eingeblendete Informationselemente der Augmented-Reality- bzw. Datenbrille, bei der die Einblendung zwischen den bereichsweise abdunkelbaren Brillengläsern und dem Auge erfolgt, durch Abdunklung eines entsprechenden Bereichs der Brillengläser mit einem dunkleren Hintergrund hinterlegt werden (1502). Hierdurch werden der Kontrast und damit die Erkennbarkeit der eingeblendeten Bilder, Texte oder Zeichen verbessert.

Dass die Hervorhebung eines Bereichs bzw. der eingeblendete Hintergrund für das Auge unscharf erscheint, ist bei der Augmented-Reality- bzw. Datenbrillenfunktionalität nicht nachteilig. Auch eine Blendschutzbrille einfacherer Bauart ohne Pupillenerkennung kann die oben genannten Augmented-Reality- bzw. Datenbrillenfunktionen aufweisen.

Die hier beschriebene Blendschutzbrille erfasst für ihre Blendschutzfunktionalität Daten, die auch zur Diagnose bestimmter Krankheiten eingesetzt werden können. Wenn die erfassten Werte auf eine Erkrankung oder eine andere Störung der Befindlichkeit des Trägers hindeuten, kann eine Benachrichtigung des Trägers, oder ein Notruf ausgelöst werden.

Die Feststellung einer Störung der Befindlichkeit des Trägers kann beispielsweise auf folgenden Daten beruhen: Unterschied der Pupillenweite beider Augen bei Lichtverhältnissen im Normalbereich, unterschiedliche Höhe der oberen Augenlider, unterschiedliche Höhe der unteren Augenlider, Veränderungen der Pupillenreaktion, Veränderungen der Hautstruktur. Hierfür kann auch eine Erweiterung der Sensorik der Blendschutzbrille vorgesehen sein, etwa ein Wärmesensor im Brillengestell, Messeinrichtungen im Brillengestell zur Erfassung des Haut- bzw. Körperwiderstands, Ausweitung des erfassten Winkels der Kameras über die bereits beschriebenen Bereiche hinaus, um mehr Daten über den Träger zu bekommen, und/oder eine Erweiterung des differenzierbaren Spektrums der Augenkameras, um im IR-Bereich die Haut- und Augentemperatur ermitteln zu können.

Medizinische Symptome, die mit diesen Daten diagnostiziert werden können sind beispielsweise folgende: Unterschied in den Pupillenweiten der Augen (Anisokorie) bei Unterschieden der Pupillenweite von mehr als 1mm, Herabhängen des oberen Augenlids (Ptosis), Fehlstellung des Augenlids (Ektropium), Horner-Syndrom, Fieber, Unterkühlung. Diese medizinischen Symptome können wiederum auf dahinterliegende gesundheitliche Probleme hindeuten: Erkrankungen des unwillkürlichen Nervensystems, Gehirntumore, Schlaganfall, Schilddrüsenkrebs, Erfrierung und vieles mehr.

Die Benachrichtigung des Trägers kann etwa über ein von dem externen Gerät ausgegebenes Signal erfolgen, oder es kann beispielsweise in einer Ecke der Brillengläser ein Bereich periodisch abgedunkelt werden; so kann auch etwa ein niedriger Ladezustand eines Akkus signalisiert werden. Dies würde auf den Träger ähnlich wie ein Blinklicht wirken, ohne die Sicht wesentlich einzuschränken. Das externe Gerät kann dann eine differenzierte Nachricht ausgeben; wenn beispielsweise eine entsprechende Applikation auf einem Smartphone gestartet wird, kann die Blendschutzbrille die ermittelten Symptome mit dem Hinweis übertragen, dass ein Arzt aufzusuchen ist. In Kombination mit einer Datenbrille, die die Einblendung von scharfem, lesbarem Text ins Gesichtsfeld ermöglicht, kann eine entsprechende Benachrichtigung direkt über diese Einblendfunktion erfolgen.

Es ist auch möglich, dass aus den ermittelten Daten ein medizinischer Notfall, wie beispielsweise ein Schlaganfall, abgeleitet wird. Um in einem solchen Notfall Hilfe herbeizuholen, kann die Blendschutzbrille um Absetzen einer Benachrichtigung an vorbestimmbare Personen oder eines Notrufs über ein mobiles Telefonnetz oder einen mobilen Internetzugang ausgestaltet sein, wobei durch eine Vernetzung mit einem Navigationssystem oder ein anderes Standortbestimmungssystem eine Angabe des Standorts erfolgen kann. Wird ein externes Gerät verwendet, das permanent beispielsweise über Nahfunk mit der Blendschutzbrille verbunden ist, können die zusätzlichen Funktionalitäten zur Positionsbestimmung und zum Notruf auch dorthin ausgelagert sein. Ist eine permanente Überwachung des körperlichen Zustands gewollt, kann die Übermittlung von geeigneten Daten über das Internet oder Nahfunk an einen vorbestimmbaren Empfänger auch dann erfolgen, wenn kein Notfall vorliegt. In Kombination mit einer Datenbrille können entsprechende Funktionalitäten gemeinsam genutzt werden.

Bei Benutzung der Blendschutzbrille beim Führen eines Kraftfahrzeugs kann die Brille Teil einer Einschlafsteuerung sein, die bei erkannten Einschlafsymptomen Sicherungs- und/oder Aufweckaktionen auslöst. Mit der erwähnten Nahfunkeinrichtung können entsprechende Daten beispielsweise an das Kraftfahrzeug übermittelt werden. Die Brille kann auch mit einer Einschlafsteuerung für andere Zwecke kombiniert werden, wie beispielsweise zum Abschalten eines Fernsehers. Eine solche Kombination ist vorteilhaft, da die hier beschriebene Brille bereits Komponenten und Funktionen beinhaltet, die für die Einschlafsteuerung mitverwendet werden kann.

Für Träger optischer Korrekturbrillen ist es wünschenswert, dass die Funktionalität der Blendschutzbrille mit optischen Brillengläsern kombiniert wird. In diesem Fall ist es vorteilhaft, bei der Berechnung der abzudunkelnden Flächen der Gläser die Lichtbrechung in den Korrekturlinsen zu berücksichtigen.

## Patentansprüche

1. Verfahren zum Reduzieren der von einem Träger einer Brille (100), deren Gläser (102) bereichsweise abdunkelbar sind, wahrnehmbaren Helligkeit mindestens eines Objekts, wobei mindestens ein Durchtrittspunkt eines Sehstrahls von einem Auge zum Objekt durch ein vor dem Auge angeordnetes Glas (102) der Brille (100) bestimmt und das vor dem Auge angeordnete Glas (102) in einem Bereich (102', 310, 311, 420, 422, 430, 432) um den Durchtrittspunkt abgedunkelt wird, **dadurch gekennzeichnet, dass** ein aktueller Pupillendurchmesser D des mindestens einen Auges ermittelt wird und dass eine Größe und/oder ein Abdunklungsgrad des abgedunkelten Bereichs (102', 310, 311, 420, 422, 430, 432) vom ermittelten Pupillendurchmesser D abhängt.

2. Verfahren nach Anspruch 1, **dadurch gekennzeichnet, dass** bei größerem Pupillendurchmesser D ein größerer Bereich (102', 310, 311, 420, 422, 430, 432) abgedunkelt wird.

3. Verfahren nach Anspruch 1 oder 2, **dadurch gekennzeichnet, dass** der Abdunklungsgrad des abgedunkelten Bereichs (102', 310, 311, 420, 422, 430, 432) von einer vorgebbaren oder automatisch anpassbaren Empfindlichkeitsfunktion abhängt.

4. Verfahren nach einem der vorhergehenden Ansprüche, **dadurch gekennzeichnet, dass** eine Größe und/oder eine Form des abgedunkelten Bereichs (102', 310, 311, 420, 422, 430, 432) von einer Größe bzw. einer Form des Objekts abhängt.

5. Verfahren nach einem der vorhergehenden Ansprüche, **dadurch gekennzeichnet, dass** eine zeitliche Veränderung des Pupillendurchmessers D erfasst wird und dass der Abdunklungsgrad des abgedunkelten Bereichs (102', 310, 311, 420, 422, 430, 432) von der zeitlichen Veränderung des Pupillendurchmessers D abhängt.

6. Verfahren nach einem der vorhergehenden Ansprüche, **dadurch gekennzeichnet, dass** der Bereich (102', 310, 311, 420, 422, 430, 432) dann abgedunkelt wird, wenn der Pupillendurchmesser D eine Anschaltschwelle Dₒₙ unterschreitet und dass der Bereich (102', 310, 311, 420, 422, 430, 432) dann nicht oder geringer abgedunkelt wird, wenn der Pupillendurchmesser D eine Abschaltschwelle D_{off} überschreitet.

7. Verfahren nach einem der vorhergehenden Ansprüche, **dadurch gekennzeichnet, dass** eine Position der Pupille (P, 1103) des mindestens einen Auges erfasst wird und dass der Durchtrittspunkt des Sehstrahls vom Mittelpunkt der Pupille (P, 1103) zum Objekt bestimmt wird.

8. Verfahren nach einem der vorhergehenden Ansprüche oder nach dem Oberbegriff von Anspruch 1, **dadurch gekennzeichnet, dass** eine Mimik des Trägers der Brille (100) erfasst wird und eine Größe und/oder ein Abdunklungsgrad des abgedunkelten Bereichs (102', 310, 311, 420, 422, 430, 432) von der Mimik abhängig ist.

9. Verfahren nach Anspruch 8, **dadurch gekennzeichnet, dass** mindestens ein Situationsparameter erfasst wird und die erfasste Mimik nur dann berücksichtigt wird, wenn der mindestens eine Situationsparameter anzeigt, dass eine Veränderung der Mimik nicht durch eine Veränderung der Situation des Trägers verursacht ist.

10. Verfahren nach einem der vorhergehenden Ansprüche, **dadurch gekennzeichnet, dass** ein erfasster zeitlicher Verlauf des Pupillendurchmessers D und/oder ein Abdunklungssignal zur Abdunklung des Bereichs (102', 310, 311, 420, 422, 430, 432) tiefpassgefiltert wird.

11. Verfahren nach einem der vorhergehenden Ansprüche, **dadurch gekennzeichnet, dass** eine Bewegung des Objekts erfasst wird und das Glas (102) zur vorausschauenden Abdunklung angesteuert wird.

12. Verfahren nach einem der vorhergehenden Ansprüche, **dadurch gekennzeichnet, dass** aufgrund des Pupillendurchmessers D eine Störung einer Befindlichkeit des Trägers erkannt und ein entsprechendes Signal an den Träger oder an eine Überwachungsinstanz übermittelt wird.

13. Verfahren nach einem der vorhergehenden Ansprüche, **dadurch gekennzeichnet, dass** ein gegenüber anderen Objekten hervorzuhebendes Objekt identifiziert wird und das Glas (102) in Bereichen um Durchtrittspunkte von Sehstrahlen zu nicht hervorzuhebenden Objekten abgedunkelt wird.

14. Blendschutzbrille, umfassend zwei elektronisch ansteuerbare, bereichsweise abdunkelbare Gläser (102), ein Gestell (101) zum Halten der Gläser (102) vor den Augen eines Trägers, eine elektronische Szenekamera (103) zum Erfassen einer von dem Träger beobachtbaren Szene, eine Pupillenerfassungseinrichtung (104) und eine Steuerungseinrichtung (107), die zum Ermitteln eines Durchtrittspunkts eines Sehstrahls von mindestens einem Auge des Trägers zu einem von der Szenekamera (103) erfassten Objekt durch ein vor dem Auge angeordnetes Glas (102) der Blendschutzbrille (100) und zum Ansteuern der Gläser (102) zum Abdunkeln eines Bereichs (102', 310, 311, 420, 422, 430, 432) um den Durchtrittspunkt ausgebildet ist, **dadurch gekennzeichnet, dass** die Pupillenerfassungseinrichtung (104) zum Erfassen eines aktuellen Pupillendurchmessers D des mindestens einen Auges des Trägers ausgebildet ist und dass die Steuerungseinrichtung (107) zum Ansteuern der Gläser zum Abdunkeln des Bereichs (102', 310, 311, 420, 422, 430, 432) des Glases (102) ausgebildet ist, wobei eine Größe und/oder ein Abdunklungsgrad des Bereichs (102', 310, 311, 420, 422, 430, 432) vom Pupillendurchmesser D abhängig ist.

15. Blendschutzbrille nach Anspruch 14, **dadurch gekennzeichnet, dass** die Blendschutzbrille (100) eine Mehrzahl von Betriebsweisen aufweist, wobei eine Betriebsweise mit verringerter Stromaufnahme vorgesehen ist.

## Claims

1. Method for reducing the brightness of at least one object perceivable by a wearer of spectacles (100), the lenses (102) of which can be darkened in regions, wherein at least one passage point of a visual line from an eye to the object through a lens (102) of the spectacles (100) that is arranged in front of the eye is determined and the lens (102) that is arranged in front of the eye is darkened in a region (102', 310, 311, 420, 422, 430, 432) around the passage point, **characterized in that** a current pupil diameter D of the at least one eye is determined, and **in that** a size and/or a degree of darkening of the darkened region (102', 310, 311, 420, 422, 430, 432) are/is dependent on the pupil diameter D determined.

2. Method according to Claim 1, **characterized in that** a larger region (102', 310, 311, 420, 422, 430, 432) is darkened in the case of a larger pupil diameter D.

3. Method according to Claim 1 or 2, **characterized in that** the degree of darkening of the darkened region (102', 310, 311, 420, 422, 430, 432) is dependent on a predefinable or automatically adaptable sensitivity function.

4. Method according to any of the preceding claims, **characterized in that** a size and/or a form of the darkened region (102', 310, 311, 420, 422, 430, 432) are/is dependent on a size and/or a form, respectively, of the object.

5. Method according to any of the preceding claims, **characterized in that** a temporal variation of the pupil diameter D is detected, and **in that** the degree of darkening of the darkened region (102', 310, 311, 420, 422, 430, 432) is dependent on the temporal variation of the pupil diameter D.

6. Method according to any of the preceding claims, **characterized in that** the region (102', 310, 311, 420, 422, 430, 432) is darkened if the pupil diameter D falls below a switch-on threshold Dₒₙ, and **in that** the region (102', 310, 311, 420, 422, 430, 432) is not darkened or is darkened to a lesser extent if the pupil diameter D exceeds a switch-off threshold D_{off}.

7. Method according to any of the preceding claims, **characterized in that** the position of the pupil (P, 1103) of the at least one eye is detected, and **in that** the passage point of the visual line from the midpoint of the pupil (P, 1103) to the object is determined.

8. Method according to any of the preceding claims or according to the preamble of Claim 1, **characterized in that** a facial expression of the wearer of the spectacles (100) is detected and a size and/or a degree of darkening of the darkened region (102', 310, 311, 420, 422, 430, 432) are/is dependent on the facial expression.

9. Method according to Claim 8, **characterized in that** at least one situation parameter is detected and the detected facial expression is taken into account only if the at least one situation parameter indicates that a change in the facial expression is not caused by a change in the situation of the wearer.

10. Method according to any of the preceding claims, **characterized in that** a detected temporal profile of the pupil diameter D and/or a darkening signal for darkening the region (102', 310, 311, 420, 422, 430, 432) are/is low-pass-filtered.

11. Method according to any of the preceding claims, **characterized in that** a movement of the object is detected and the lens (102) is driven for predictive darkening.

12. Method according to any of the preceding claims, **characterized in that** a disturbance of the wearer's state is identified on the basis of the pupil diameter D and a corresponding signal is communicated to the wearer or to a monitoring entity.

13. Method according to any of the preceding claims, **characterized in that** an object to be highlighted relative to other objects is identified and the lens (102) is darkened in regions around passage points of visual lines to objects that are not to be highlighted.

14. Anti-glare spectacles, comprising two electronically drivable lenses (102) that can be darkened in regions, a frame (101) for holding the lenses (102) in front of a wearer's eyes, an electronic scene camera (103) for detecting a scene that can be observed by the wearer, a pupil detection device (104) and a control device (107), which is designed for determining a passage point of a visual line from at least one eye of the wearer to an object detected by the scene camera (103) through a lens (102) of the anti-glare spectacles (100) that is arranged in front of the eye, and for driving the lenses (102) for darkening a region (102', 310, 311, 420, 422, 430, 432) around the passage point, **characterized in that** the pupil detection device (104) is designed for detecting a current pupil diameter D of the at least one eye of the wearer, and **in that** the control device (107) is designed for driving the lenses for darkening the region (102', 310, 311, 420, 422, 430, 432) of the lens (102), wherein a size and/or a degree of darkening of the region (102', 310, 311, 420, 422, 430, 432) are/is dependent on the pupil diameter D.

15. Anti-glare spectacles according to Claim 14, **characterized in that** the anti-glare spectacles (100) have a plurality of operating modes, wherein an operating mode with reduced power consumption is provided.

## Revendications

1. Procédé de réduction de la luminosité d'au moins un objet perceptible par un porteur de lunettes (100), dont les verres (102) peuvent être localement assombris, dans lequel on détermine au moins un point de traversée d'un rayon optique d'un oeil à, l'objet à travers un verre (102) des lunettes (100) disposé devant l'oeil et on assombrit le verre (102) disposé devant l'oeil dans une région (102', 310, 311, 420, 422, 430, 432) entourant le point de traversée, **caractérisé en ce que** l'on détermine un diamètre de pupille actuel D dudit au moins un oeil et **en ce que** la grandeur et/ou un degré d'assombrissement de la région assombrie (102', 310, 311, 420, 422, 430, 432) dépend du diamètre de pupille déterminé D.

2. Procédé selon la revendication 1, **caractérisé en ce que** pour un plus grand diamètre de pupille D, on assombrit une plus grande région (102', 310, 311, 420, 422, 430, 432).

3. Procédé selon la revendication 1 ou 2, **caractérisé en ce que** le degré d'assombrissement de la région assombrie (102', 310, 311, 420, 422, 430, 432) dépend d'une fonction de sensibilité prévisible ou adaptable automatiquement.

4. Procédé selon l'une quelconque des revendications précédentes, **caractérisé en ce qu'**une grandeur et/ou une forme de la région assombrie (102', 310, 311, 420, 422, 430, 432) dépend d'une grandeur et/ou d'une forme de l'objet.

5. Procédé selon l'une quelconque des revendications précédentes, **caractérisé en ce que** l'on détecte une variation temporelle du diamètre de pupille D et **en ce que** le degré d'assombrissement de la région assombrie (102', 310, 311, 420, 422, 430, 432) dépend de la variation temporelle du diamètre de pupille D.

6. Procédé selon l'une quelconque des revendications précédentes, **caractérisé en ce que** l'on assombrit la région (102', 310, 311, 420, 422, 430, 432) lorsque le diamètre de pupille D descend en dessous d'un seuil de connexion Dₒₙ et **en ce que** l'on n'assombrit pas ou moins la région (102', 310, 311, 420, 422, 430, 432) lorsque le diamètre de pupille D dépasse un seuil de coupure D_{off}.

7. Procédé selon l'une quelconque des revendications précédentes, **caractérisé en ce que** l'on détecte une position de la pupille (P, 1103) dudit au moins un oeil et **en ce que** l'on détermine le point de traversée du rayon optique du point central de la pupille (P, 1103) à l'objet.

8. Procédé selon l'une quelconque des revendications précédentes ou selon le préambule de la revendication 1, **caractérisé en ce que** l'on détecte une mimique du porteur des lunettes (100) et une grandeur et/ou un degré d'assombrissement de la région (102', 310, 311, 420, 422, 430, 432) dépend de la mimique.

9. Procédé selon la revendication 8, **caractérisé en ce que** l'on détecte au moins un paramètre de situation et on tient compte de la mimique détectée uniquement lorsque ledit au moins un paramètre de situation indique qu'un changement de la mimique n'est pas causé par un changement de la situation du porteur.

10. Procédé selon l'une quelconque des revendications précédentes, **caractérisé en ce qu'**une évolution temporelle détectée du diamètre de pupille D et/ou un signal d'assombrissement pour l'assombrissement de la région (102', 310, 311, 420, 422, 430, 432) est filtré(e) au filtre passe-bas.

11. Procédé selon l'une quelconque des revendications précédentes, **caractérisé en ce que** l'on détecte un mouvement de l'objet et on commande le verre (102) en vue de l'assombrissement anticipé.

12. Procédé selon l'une quelconque des revendications précédentes, **caractérisé en ce que** l'on reconnaît une perturbation de la sensibilité du porteur en se basant sur le diamètre de pupille D et on transmet un signal correspondant au porteur ou à une instance de surveillance.

13. Procédé selon l'une quelconque des revendications précédentes, **caractérisé en ce que** l'on identifie un objet à mettre en évidence par rapport à d'autres objets et on assombrit le verre (102) dans des régions entourant des points de traversée de rayons optiques dirigés vers des objets à ne pas mettre en évidence.

14. Lunettes anti-éblouissement, comprenant des verres (102) pouvant être localement assombris par commande électronique, une monture (101) pour maintenir les verres (102) devant les yeux d'un porteur, une caméra panoramique électronique (103) permettant de détecter une scène observable par le porteur, un dispositif détecteur de pupille (104) et un dispositif de commande (107), qui est configuré pour déterminer un point de traversée d'un rayon optique depuis au moins un oeil du porteur à un objet détecté par la caméra panoramique (103) à travers un verre (102) des lunettes anti-éblouissement (100) disposé devant l'oeil et pour commander les verres (102) en vue de l'assombrissement d'une région (102', 310, 311, 420, 422, 430, 432) entourant le point de traversée, **caractérisées en ce que** le dispositif détecteur de pupille (104) est configuré pour détecter un diamètre de pupille actuel D dudit au moins un oeil du porteur et **en ce que** le dispositif de commande (107) est configuré pour commander les verres en vue de l'assombrissement de la région (102', 310, 311, 420, 422, 430, 432) du verre (102), dans lequel la grandeur et/ou un degré d'assombrissement de la région (102', 310, 311, 420, 422, 430, 432) dépend du diamètre de pupille D.

15. Lunettes anti-éblouissement selon la revendication 14, **caractérisées en ce que** les lunettes anti-éblouissement (100) présentent une multiplicité de modes de fonctionnement, dans lequel il est prévu un mode de fonctionnement avec une consommation électrique réduite.
